# EUROPEAN PATENT SPECIFICATION

(11) **EP 3 893 760 B1**
(45) Date of publication and mention of the grant of the patent: **18.03.2026**
(21) Application number: 19813810.9
(22) Date of filing: 03.12.2019
(51) Int. Cl.: A61B 10/04, A61B 10/00, A61B 10/02, A61B 10/06

(54) **A TISSUE SPECIMEN COLLECTOR FOR USE IN BIOPSY**
GEWEBEPROBENENTNAHMEVORRICHTUNG FÜR BIOPSIE
COLLECTEUR D'ÉCHANTILLON DE TISSU DESTINÉ À ÊTRE UTILISÉ DANS UNE BIOPSIE

(30) Priority: 14.12.2018 SE 1851593
(43) Date of publication of application: 20.10.2021
(73) Proprietor: Multi4 Medical AB, 553 13 Jönköping (SE)
(72) Inventor: BRAKHYA, Ronny, 56135 Huskvarna (SE); ULVEGARD, Hannes, 55447 Jönköping (SE); AXELSSON, Robert, 56391 Gränna (SE); ALTGÄRDE, Noomi, 56151 Huskvarna (SE); MELLE HANNAH, Miden, 55313 Jönköping (SE)
(74) Representative: AWA Sweden AB
(86) International application number: PCT/EP2019/083409
(87) International publication number: WO 2020/120215

(56) References cited:
- WO-A1-2018/088892
- JP-A- 2000 271 128
- US-A- 5 526 822
- US-A1- 2004 068 291
- US-A1- 2005 049 633
- US-A1- 2017 160 169

## Description

The present invention relates to a tissue specimen collector for collecting tissue specimens during a biopsy procedure.

The tissue specimen collector is especially for use with an endosurgical biopsy device that is adapted to be inserted via an endoscope, such as a cystoscope, to take tissue specimens from the urinary bladder.

Urinary bladder cancer is one of the most expensive forms of cancer in Sweden and internationally. In the United States estimated costs are about 140,000 dollars per newly diagnosed person. Every year there are about 2,400 new persons in Sweden diagnosed with urinary bladder cancer. In the past 20 years, the number of cases has increased by a total of 35 percent and urinary cancer affects men three times as often as women. Cancer in the urinary bladder and urinary tract is the third most common form of cancer in men in Sweden.

About 70% of patients have a superficial urinary bladder cancer form restricted to the mucosa. This means that these patients can be cured, but unfortunately there is a risk of recurrence and of residual tumor. About two thirds of patients need a new surgery. It is not uncommon for one and the same patient to undergo several operations, sometimes only a few months after the first. There will be many regular visits to the urologist to discover recurrence early so that the cancer does not grow.

Cystoscopy is the primary diagnostic modality for the diagnosis of bladder carcinoma. A conventional cystoscope normally has two ports, an optical port that permits to see inside the bladder and an instrument port for insertion of various devices. The endosurgical biopsy device of the present invention is of the kind to be inserted via the instrument port of an endoscope.

During cystoscopy, when there is suspicion of cancer in the urinary bladder, the urologist generally obtains multiple bladder tissue specimens, typically using a punch biopsy forceps, from various locations in the bladder to help establish the diagnosis and to determine the extent of a potential tumor. Generally tissue specimens are taken from any areas of abnormal-looking urothelium and from suspected tumor areas, but typically from trigone, bladder dome, and right, left, anterior and posterior bladder walls. At the time of the procedure, the bladder is lavaged with fluid. Prior to tissue acquisition during a transurethral bladder biopsy using a flexible cystoscope local anesthesia can be made at the sample site or anesthesia is obtained locally by instilling anesthetic agents into the bladder. Following tissue acquisition, the biopsy site is cauterized for hemostasis, and the procedure is repeated. Because a conventional cystoscope only has one working channel for the various devices needed during the surgical procedure, each of these various devices is moved in and out of the working channel each time a tissue specimen is taken from the bladder. Since biopsies are taken today by pulling out the instruments for each sample, the risk of contamination of samples cannot be overlooked. Mixing up samples and wrong labeling may cause series consequences. Yet a problem is that each tissue specimen is transferred to a vial of its own whereby valuable knowledge of its position and orientation at the target tissue becomes lost.

The failure to appreciate and standardize thickness of tissue specimen, orientation of the tissue specimen, time until fixation, the further manipulation steps, potential cross-contamination between specimens, contamination from the environment, and the fixation process itself, are all parameters involved for the analyst to make the best possible histomorphologic diagnosis on the tissue specimen.

In case the suspect organ is the urinary bladder the biopsy procedure recommends a total of seven biopsies to be performed of masses and/or tissue. The tissue specimens are transferred one after another to individual vials properly labeled prior to the biopsy procedure. Information on the label includes identification data of the patient and optionally the origin and the exact biopsy site of the relevant tissue specimen, or other identification that links the vial to the specific origin of tissue specimen.

WO 2018/088892 discloses a storage container for individual spare polyp collectors that slides in a housing. The polyp collectors are stored until they are ready for use to enter a collecting space. The collectors are separate containers, and are not arranged in a commom rack. The filled polyp collector is been released and dropped into the bottle by operating a slider that works crosswise the axis of the storage container, not longitudinally, and serves to close the flow path from a sampling instrument to the sample-containing polyp collector, and open a flow path to a bottle thereby allowing the filled polyp collector to drop into said bottle. This procedure is repeated for each polyp collector.

US 2017/160169 discloses a simple device consisting of a tray having two specimen wells and is arranged to reciprocate in an out of a sleeve. A first specimen well is positioned within the interior cavity of the sleeve and in a suction pathway, and the second specimen well is positioned out of the suction pathway exterior to the interior cavity of the sleeve. Upon collection of the first specimen in the first specimen well, the tray is moved to place the second well into the suction pathway, and the first well is placed out of the suction pathway exterior of the cavity. The first specimen is taken out of the well and a second specimen collected in the second well. This action can be repeated and the wells are reused and exposed to contamination during manual transfer.

US 2004/068291 discloses a tissue slice trap body that slides in a U-shaped support. The trap body is a strip having traps. severed from the trap body at the through slits. When a trap holds a tissue slice it is by manual interaction severed from the strip, an put directly into a sample bottle that contains a tissue fixing agent, so that the tissue slice is immersed in the tissue fixing agent. This procedure must be repeated for each tissue slide so the tissue slice is exposed to contamination during manual transfer.

US patent no. 5,526,822 relates to an automated biopsy device having a piercing needle with a receiving port to trap tissue prior to cutting. A tissue sample cassette provides storage for the samples as well as a means for coding and decoding the location from which the samples were obtained. A prolapsed tissue sample is severed from the main tissue mass by the advancement of an cannular inner cutter along its axis within a hollow outer piercing needle past a tissue receiving port. The tissue sample is packed into the cannular cutter as it moves forward against and rests inside said cannular inner cutter. The cannular inner cutter is then withdrawn holding the tissue sample in the cannular inner cutter by friction with the inner walls of the cannula and by the suction created by a vacuum source and delivered by a tubular knock out pin. The tissue sample is deposited in a tissue sample cassette by positioning the tubular knock out pin coaxially within the cannular inner cutter and the hollow outer piercing needle such that a distal end of the tubular knock out pin is near the proximal end of the tissue containment chamber. As the cannular inner cutter is withdrawn through the tissue containment chamber, the tissue sample is stopped within the tissue containment chamber by the distal end of the tubular knock out pin. The final release of the tissue sample from the tubular knock out pin into the tissue containment chamber takes place by turning the vacuum source off thereby releasing the tissue sample from the distal end of the tubular knock out pin whereby the tissue sample is deposited within a chamber of the tissue sample cassette.

This known device depends on contact between the tissue sample and rigid instruments, such as the knock out pin and the cannular cutter, to place said tissue sample in the tissue compartment, which contact can be detrimental to the quality of the sample, and even destroy the sample. Morever the same rigid instruments are used for pushing each new tissue sample forward into a subsequent chamber of the tissue sample cassette, thus cells sticking to the rigid instruments may contaminate the subsequent individual samples because the rigid instruments are not cleaned between samplings.

It has been realized that there is an increasing need within the art for improvements in collecting tissue specimens, in particular in collecting tissue specimens using endosurgical biopsy devices via endoscopes, such as a flexible endoscope. The flexible endoscope may be a cystoscope and the organ be the urinary bladder.

In a main aspect of the present invention is provided a tissue specimen collector of the kind mentioned in the opening paragraph.

In a further aspect of the present invention is provided a tissue specimen collector of the kind mentioned in the opening paragraph for use with an endosurgical biopsy device via an endoscope while the endosurgical biopsy device is still inserted in the endoscope's working channel, so that the endosurgical biopsy device need not be moved in and out of said working channel many times for completing the required numbers of biopsies in a non-destructive and safe manner.

In yet an aspect of the present invention is provided a tissue specimen collector of the kind mentioned in the opening paragraph with an improved operative functionality.

In yet an aspect of the present invention is provided a tissue specimen collector of the kind mentioned in the opening paragraph and a multi-functional endosurgical biopsy device for successive tissue sample taking without inter-sample contamination.

In yet an aspect of the present invention is provided a multi-functional endosurgical biopsy device for taking and collecting several tissue specimens, in particular for taking tissue specimens from the urinary bladder, and being adapted for transferring tissue specimens directly into a tissue specimen collector of the kind mentioned in the opening paragraph.

The novel and unique whereby these and other aspects are achieved according to the present invention consists in that the tissue specimen collector is adapted for collecting tissue specimens during a biopsy procedure, wherein the tissue specimen collector is a multi-chamber collector having
- a main collector housing extending along a longitudinal axis,
- a collector port adapted to connect to a supply of tissue specimens, wherein the multi-chamber collector has
- a collector rack having a series of adjacent chambers for accommodating tissue specimens, which collector rack is arranged lengthwise displaceable inside the main collector housing to move translatory inside said main collector housing by actuating means for moving the collector rack lengthwise along the longitudinal axis of the main collector housing to position said chambers one after another in fluid communication with the collector port wherein said collector housing is configured to keep all of the tissue specimens in a closed environment simultaneously, and said main collector housing has a collector port adapted to connect to a supply of tissue specimens.

The tissue specimen collector is a multi-chamber collector that directly collects a resected, acquired tissue specimen in a dedicated chamber at the inlet of the main collector housing, such as upon exit of a sample port from the device used to acquire the tissue specimen. Such a device can e.g. be the endosurgical biopsy device described below and operated via the working channel of an endoscope, e.g. a flexible cystoscope in case the organ wherefrom the tissue specimens are to be taken is the urinary bladder.

The novel tissue specimen collector advantageously receives and accommodates the tissue specimens in the order they are resected, preferably collecting the tissue specimen in a known orientation in the dedicated chamber in view of the orientation at the target site wherefrom the tissue specimen is taken. Within the scope of the present invention the term "dedicated chamber" means that each chamber collect a tissue specimen in of a tissue specimen acquisition series. A chamber is then allocated to collect a specific tissue specimen of the series. The terms "chamber" and "dedicated chamber" are used herein interchangingly in the context of the collector rack.

To collect tissue specimens one after another in dedicated chambers the tissue specimen collector has a collector rack arranged to move translatory inside the main collector housing by actuating means for moving the collector rack lengthwise along the longitudinal axis of the main collector housing to position said dedicated chambers one after another in fluid communication with the collector port, and with said collector port being aligned below a sample port, such as an appropriate opening, outlet, or similar discharge means, from the endosurgical biopsy device.

In this way the tissue specimen collector is configured to collect several tissue specimens one after the other in a continued sequence without removing an endosurgical biopsy device from an endoscope used during the tissue specimen acquisition procedure.

Alignment of the collector port with a sample port of the endosurgical biopsy device, and with consecutive chambers of the collector rack, constitutes a simple manner of making the order that the tissue specimens are resected and acquired to correspond to the order that the dedicated chambers are arranged below said combined and aligned ports. Once a dedicated chamber has been filled with a tissue specimen a new dedicated chamber is moved in liquid communication with the aligned collector port and sample port, and said chamber is thus ready to receive the next tissue specimen. This procedure is continued until the full tissue specimen sampling map has been completed, in which case all or the intended number of dedicated chambers has received a tissue specimen. The collector rack may have extra channels. For a urinary bladder biopsy the collector rack may typically have seven or more dedicated chambers.

The dedicated chambers may be sized and dimensioned so that once a tissue specimen is arrived and accommodated in said chamber, said tissue specimen cannot move around. Since the tissue specimen has little opportunity to shift orientation out of a closed biopsy cup and during its travel inside a narrow tube channel, the risk that the tissue specimen change orientation is substantially reduced compared to conventional techniques, where the surgeon's assistant simply collects the tissue specimens one after another from the end effector, and manually drops them in separate, labeled vials where the tissue specimen flows freely around.

Using the tissue specimen collector of the present invention only one labeling action is required in that all tissue specimens from one procedure are collected in a single multi-chamber cassette or cartridge, namely the collector rack, that inherently informs of the biopsy site, e.g. by having patient ID, a number, or other information that allocates a tissue specimen in a dedicated chamber to a site on the biopsy procedure map. The entire collector rack can be send directly to histology analysis for establishing a diagnosis.

The collector port may advantageously extend into a coupling piece protruding from the main collector housing to easily be snapped on, or otherwise connected to, an outlet of the endosurgical biopsy device, such as a sample port, so that the tissue specimen, from being resected to being collected, remains in a closed environment. To that aspect the coupling piece may be adapted for providing fluid coupling to the sample port of an endosurgical biopsy device.

One way of moving the tissue specimen between an end effector, such as a forceps, in the direction of the main collector housing is flushing with a physiological acceptable liquid, such as saline or glycine, or suction, along the tube, which is the part of the endosurgical device that has been inserted into the working channel of the endoscope, whereby the tissue specimens can be collected without extra external human interaction other than the interaction actuated by the surgeon to e.g. flush, suck or aspirate the tissue specimen from the biopsy site into each prearranged receptacle, namely a chamber of the collector rack.

A further advantage of flushing the tissue samples out of the end effector into the tissue specimen is that the risk of inter-sample contamination is eliminated, or at least substantially reduced.

In case that the procedure to move the tissue specimen out of the endosurgical instrument is flushing or aspirating a tube the main collector housing may have a liquid outlet to discharge liquid accompanying the tissue specimen entering the tissue specimen collector. Such a liquid outlet may be connected to a conduit leading to a reservoir for collecting the flushing liquid. In case of suction instead of flushing the main collector housing may also have a vent or valve for equalizing pressure prior to the next biopsy.

The means for moving the collector rack lengthwise along the longitudinal axis of the main collector housing may in a first embodiment of a tissue specimen collector simply be a screw rod with an exterior thread, which screw rod serves to be screwed deeper and deeper inside a threaded hole or bore in the collector rack, or *vice versa,* thereby carrying the collector rack along. The screw rod may have a knob at a free end facing away from the collector rack, which knob is rotated to screw the rod in and out of the hole or bore.

Other arrangements to stepwise, and in sequence, fix the position of the dedicated chambers of the collector rack below the sample port of the endosurgical biopsy device may be a ratchet mechanism, for example a ratchet mechanism consisting of at least a linear rack on the collector rack and a pawl, such as a spring-biased pawl, that stepwise engages the linear rack to displace the collector rack in relation to the main collector housing.

The main collector housing may be a single housing or consist of at least two housing parts, which at least two housing parts advantageously can be detachably assembled in order to be able to take the collector rack, and thus also the acquired tissue specimens, out for analysis of the tissue specimens. Two housing parts can be joined end to end using a seal or gasket to make the assembled main collector housing leak-proof and airtight to be able to resist flushing and/or suction without loosing pressure.

Yet an alternative embodiment of an arrangement to stepwise, and in sequence, fix the position of the dedicated chambers of the collector rack below the sample port of the endosurgical biopsy device may be opposite male/female stepwise engaging/disengaging components at suitable distances along the collector rack and main collector housing instead of the ratchet mechanism, which distances can correspond to or be multiples of e.g. the distance between adjacent chamber.

The collector rack may expediently be configured to allow liquid to pass through, e.g. by having a plurality of through-holes or canals extending from inside a chamber to the exterior of the collector rack and/or through-openings that extend through common walls of adjacent chambers and/or through exterior walls of the collector rack. In another embodiment the collector rack can be made of a filtering material, be porous, or having interstices through which liquid may pass into the main collector housing and out through the liquid outlet of the main collector housing, e.g. to be collected in a reservoir.

A suitable endosurgical biopsy device that is especially adapted for use with the tissue specimen collector of the present invention can be an endosurgical biopsy device comprising:
- a tube that has at least a first lengthwise extending channel for supplying a liquid to an end effector, a second lengthwise extending channel for removing matter from or at the end effector, and a third lengthwise extending channel for accommodating an electrical wire connected to the end effector to apply current to perform diathermy, wherein
- the end effector is provided at a distal tube end of the tube, and
- the tissue specimen collector described above is provided in fluid communication with a proximal tube end of the tube.

By using the term "diathermy" in the context of the present invention is meant that the end effector, e.g. a forceps having opposite jaws, of the endosurgical biopsy devices suited for use with the tissue specimen collector of the present invention are adapted to generate heat in organ tissue by high-frequency electromagnetic currents. The high-frequency electromagnetic currents pass through tissue and make a precise surgical incision like a scalpel blade, whereby the surgical diathermy provides fine, precise incisions and tissue specimens.

The endosurgical biopsy device suited for use with the tissue specimen collector may comprise
- an effector sleeve surrounding the tube at least at the distal tube end of said tube, and being arranged to reciprocate at least at the distal tube end of the tube,
- a device operating part comprising a slide rail assembly, which slide rail assembly comprises at least a main slide guide body that accommodates an end effector slider and a junction slider,
- the main slide guide body has a guideway with a proximal guide end and an opposite distal guide end that receives the tube,
- the end effector slider is adapted to reciprocatingly slide in the guideway at the distal guide end, and is connected to the effector sleeve to operate said effector sleeve in relation to the end effector, and
- the junction slider is adapted to reciprocatingly slide in the guideway between the proximal guide end and the end effector slider, which junction slider is connected to the tube to arrange at least the first lengthwise extending channel, the second lengthwise extending channel and the third lengthwise extending channel of the tube in communication with the end effector.

The end effector slider may be adapted to slide in the guideway to operate the effector sleeve in relation to the end effector by being connected to the effector sleeve via a reciprocatable connecting means surrounding the tube, such as a coil member on the outside of the tube, or a reciprocatable connecting means inside the tube, **e.g.** an effector wire inside one of the lengthwise extending channels.

The junction slider is slidingly arranged in relation to the guideway to move the end effector at the distal tube end out of the working channel to meet the target tissue.

The junction slider may have at least one of
- an inlet port in fluid communication with the first lengthwise extending channel for delivering a flushing liquid at and/or to the end effector,
- a diathermy port in communication with the third lengthwise extending channel, and/or
- a sample port in communication with the second lengthwise extending channel for coupling into liquid communication with the tissue specimen collector.

Within the context of the present invention the term "proximal" means closest to something and the term "distal" means farthest from something. As an example the proximal tube end is the end closest to the operating end of the endoscope when in use, and the distal tube end is the end closest to the tip of the operating part of the endoscope. The term "proximal" are thus used herein in consistent manner to indicate when a structural feature is closest to the operating part of the endosurgical biopsy device and to the operating part of the endoscope. The term "distal" means opposite "proximal".

The tube may have the effector sleeve at its distal tube end, which effector sleeve is adapted to reciprocate along the length of the tube to change configurations of the end effector, such as to open and close opposite jaws of the end effector. The effector sleeve can be a single unit in form of an electrically insulated sleeve extending the whole length from the end effector to the junction slider. The effector sleeve can also be operatively connected to the end effector slider via another tubular member, such as e.g. the coil member surrounded by an electrically insulating tube.

Irrespective of how the effector sleeve is configured and connected to the end effector slider it is lengthwise displaceable by sliding the end effector slider to move the effector sleeve along the tube. This way the end effector can be alternating arranged in a sampling position, where the end effector is at least partly exposed from said effector sleeve, and a confined position, where the end effector is located at least partly inside the effector sleeve.

The junction slider serves to move the tube with the end effector surrounded by the effector sleeve out of the distal opening of the endoscope, thus to move the end effector in the desired position at the target site. When the end effector slider is pulled towards the proximal guide end it retracts the effector sleeve and exposes the end effector which opens the jaws. Moving the end effector slider forward again towards the distal guide end pushes the effector sleeve forward to close the jaws around the tissue specimen.

So if the end effector is of the forceps kind having opposite jaws, said opposite jaws can be moved apart by operating the end effector slider, e.g. by pulling or pushing at the effector sleeve, e.g. by means of an effector wire extending between the end effector and the end effector slider, or by means of a reinforcing coil around the tube, which reinforcing coil can be secured at opposite ends to the effector sleeve and the end effector slider, respectively.

In the position of the end effector where it is free of the effector sleeve, the jaws can grasp and cut off a tissue specimen by application of diathermy. When the jaws are to be closed again the end effector slider is operated to pull the end effector back again inside the effector sleeve. The tissue specimen is confined between the closed jaws.

In the alternative to reciprocating the effector sleeve the end effector can be reciprocated in and out of the effector sleeve.

When the end effector is fully, or substantially fully, inside the effector sleeve, the jaws of an end effector in form of a forceps is closed to define a closed biopsy cup. The effector sleeve effectively seals any gaps around the closed jaws of the end effector.

The effector sleeve may e.g. be a steel sleeve, a polymeric sleeve, and even be slightly elastic, but not more elastic than it can keep the jaws together. It may be preferred that the endosurgical biopsy device has a firm fit between an interior surface of the effector sleeve and the exterior surface of the tube to prevent liquid from flowing externally to the tube along a gap between said surfaces towards the slide rail assembly. In the alternative a gasket can be used as a seal against said surface of the tube. Such fit or sealing are of the kind that allows the effector sleeve to reciprocate along the tube. Typically the end effector and/or a further tubular member, which are used to couple the end effector and the junction slider in operative communication, are in sliding contact.

Advantageously the tubular member, such as a coil member that connects the effector sleeve with the end effector slider, may also serve as the reinforcing member of the tube, so as to provide the endosurgical biopsy device with required stiffness and structure to be guided along the working channel of the endoscope without coiling or kinking. The effector sleeve can be made of a material that does not take away the bending properties of e.g. a bendable tube or tubular member, be a tubular member having insulation properties, etc.. In embodiments having a long effector sleeve extending from the end effector to the junction slider the reinforcing member may serve as the effector sleeve. The reinforcing member or other tube, such as a coiled member or spiral member, can then advantageously be used around the tube to make the tube sufficiently rigid to be moved in and out of the working channel of the endoscope but still be bendable and maneuverable. When using e.g. a reinforcing spiral member or coiled member to add support to the tube, this member may advantageously be encapsulated by an exterior heat-shrink tubing or sheath. The heat-shrink tubing advantageously seals and insulates the endosurgical biopsy device along its length, **e.g.** to prevent it from letting out flushing fluid, but also to make it easy for the endosurgical biopsy device to be passed through the working channel due to low friction, and so that the tube cannot get in electrical contact with the working channel.

The exterior heat-shrink tubing or sheath may be used to combine effector sleeve and reinforcing member end-to-end.

The junction slider may communicate with the first, the second and the third lengthwise extending channels inside the tube.

Liquid to flush the target wherefrom tissue specimens are to be acquired is conveniently provided into the first lengthwise extending channel via a flow channel of the junction slider. Then diathermy is applied to the tissue by applying current from a diathermy generator to the end effector via the electrical wire in the third lengthwise extending channel. The electrical wire extends inside the third lengthwise extending channel of said tube and ends at the junction slider of the slide rail assembly.

In the embodiment wherein the main collector housing of the tissue specimen collector of the present invention has a collector port for fluidly coupling to the sample port of the junction slider of the endosurgical biopsy device, optionally by means of an inserted coupling piece, said main collector housing may also have a liquid outlet to discharge liquid entering the main collector housing via the collector port from the sample port, thus from the second lengthwise extending channel during ejecting of the tissue specimen from the biopsy cup by flushing or suction.

A dedicated chamber of the collector rack can easily be aligned below the collector port, and thus below the coupling piece coupled to the sample port, to catch and receive a tissue specimen in said chamber. Surplus of liquid used to force the tissue specimen out of the second lengthwise extending channel, through the sample port, and via the coupling piece, through the collector port and into the chamber of the collector rack, can flow out of the tissue specimen collector via the liquid outlet to be collected in a reservoir or container of any suitable kind, optionally for further histology analysis, safe depositing or safe discharge.

Any of the lengthwise extending channels can be utilized to guide the electrical wire provided the electric wire is insulated to not come into contact with fluid inside any lengthwise extending channel.

In the embodiment utilizing an effector wire for operating the effector sleeve or the end effector, such an effector wire may be positioned in the first or second lengthwise extending channel.

An end effector may have opposite jaws pivotable arranged to diverge from a longitudinal axis of the end effector in a condition when the end effector is at least partly outside the effector sleeve so that the jaws can span a large tissue specimen. The jaws become closed when the end effector is retracted inside the effector sleeve or when the effector sleeve is moved forward whereby the jaws define a closed biopsy cup. After application of diathermy the gently resected tissue specimen is confined between the jaws inside the biopsy cup, and the first lengthwise extending channel and the second lengthwise extending channel of the tube become in liquid communication at the distal tube end so that when the first lengthwise extending channel is flushed the tissue specimen is forced into the second lengthwise extending channel and delivered to the junction slider that couples to the tissue specimen collector wherein said tissue specimen can be collected in a chamber.

Two opposite diverging jaws may e.g. open about 5 mm so that large tissue specimens can be obtained, and due to using diathermy these tissue specimens have clean cutting edges. Frying and burning of said edges are reduced, or even eliminated.

Besides facilitating application of resecting clean, rather large tissue specimens by application of diathermy, another huge advantage of using an endosurgical biopsy device provided with a tissue specimen collector is that the orientation of a resected tissue specimen is kept under control to a higher degree than in known biopsy procedures. A tissue specimen resected by application of diathermy can be kept confined between the jaws of the end effector in substantially same orientation as it is taken from the target tissue. The tissue specimen substantially fills the closed biopsy cup so that it cannot shift position and orientation, and the chamber of the tissue specimen collector may be designed with similar dimension and similar shape so that the tissue specimen does not turn around in a chamber once received. So a tissue specimen collector may advantageously be designed with chambers matching and correlated to the size and shape of the tissue specimens to be acquired by a given end effector and a given tube.

When e.g. a flushing liquid is injected under pressure to the first lengthwise extending channel from the junction slider the tissue specimen is forced into the second lengthwise extending channel while keeping its orientation. So it is attempted that when the tissue specimen reaches the proximal end of the tube its orientation is known. The tissue specimen can then travel from the junction slider in a known orientation into a chamber of the tissue specimen collector, thereby providing valuable diagnostic information of cellular architecture of the tissue specimen in view of having knowledge of the site specific origin and orientation of the tissue specimen. This improved ability to control orientation ensures a high level of specificity, highly reliable diagnosis, and permits a facilitated assessment of the relevance of results in relation to clinical and anatomic normal and abnormal features of the target tissue related to the tissue specimen in question.

The second lengthwise extending channel is thus selected to have a diameter that allows for gentle expelling and transfer of the tissue specimen by simple flushing, preferably under pressure, aspiration or suction using vacuum, and so that the tissue specimen is not swirled around and looses orientation. By careful dimensioning of biopsy cup and diameter of the second lengthwise extending channel the possible movements of the tissue specimen can be restricted to a very high degree, or eliminated fully.

Known forceps devices on the market that use diathermy cause the tissue specimen to be destroyed by the heat, and that the tissue specimen stick between the opposite cutting edges of the opposite jaws, so that the tissue specimen has to be manually removed from the forceps. When using diathermy for the endosurgical biopsy device used in the context of the present invention the tissue specimen is not severely damaged because the tissue specimen so easily releases from the organ and from the jaws, which jaws advantageously can be insulated exteriorly, and optionally due to the instant cooling of the jaws initiated by the flushing fluid.

The slide rail assembly of a suitable endosurgical biopsy device may advantageously further have a needle slider reciprocatingly disposed in the guideway in front of the junction slider at the proximal guide end, which needle slider may have a needle or a nozzle secured thereto, which needle or nozzle may be reciprocatingly arranged inside a fourth lengthwise extending channel of the tube between a first needle position in which the needle or nozzle is in a retracted position and a second needle position in which the needle or nozzle is exposed from the end effector, **e.g.** between open jaws of the end effector.

In the biopsy procedure the needle or nozzle may serve for application of e.g. a local anesthetic, other medicament or solution at one or more target sites or local areas, **e.g.** target sites or local areas wherefrom a tissue specimen is to be acquired by resecting. Injecting a liquid into the tissue areas to be resected expands said tissue area thereby displaying the tissue to both the fiber optics of the endoscope and offering the relevant tissue as an easy accessible target for the operator of the endosurgical device of the present invention to use the end effector. This technology of expanding tissue prior to sampling has not been used extensively because it requires at least two additional insertions and retractions of instruments of the working channel of the endoscope per tissue specimen.

The jaws of one embodiment of an end effector may in the closed configuration enclose the needle tip of the needle or the nozzle tip of the nozzle entirely. So when the biopsy cup is closed no parts of the nozzle or needle is outside said biopsy cup.

In the alternative embodiment one of the opposite jaws of the end effector has an opening configured to expose a needle or a nozzle when the jaws are closed. When the biopsy cup is closed after having been open, and the needle or nozzle having been exposed to apply anesthetics to the target, the needle or nozzle may reciprocate back inside the fourth lengthwise extending channel of the tube so that the opening is closed and plugged sealingly by the needle or nozzle.

The endosurgical biopsy device of the present invention can however also advantageously be used without general anesthesia, e.g**.** on an out-patient or for a patient at the receiving ward. The surgeon may take his decision as to anesthesia at any stage during the biopsy procedure without causing the patient inconvenience. The endosurgical biopsy device allows the surgeon to take various choices and to take several tissue specimens directly from the suspicious target area without having to repeat inserting a series of different tools into the working channel of the endoscope for each choice and taking of a tissue specimen. Cancer cells can be destructed by application of diathermy while the endosurgical biopsy device is still inside the patient. The patient can go home immediately afterwards, needs no catheter, nor fasting before the surgical biopsy procedure.

A reservoir of flushing liquid can easily be connected to the inlet port of the junction slider in various ways. One way is to insert into the inlet port a stepped tapered connector coupled to a liquid supply tube, in which case the inlet port serves as a female coupling component. Instead the liquid supply tube can be mounted on the inlet port, in which case the inlet port serves as a male coupling component.

The electrical wire may extend out of the second lengthwise extending channel at the proximal tube end and into the diathermy port of the junction slider to create an electrical contact to the diathermy generator. Such electrical contact can be obtained in a very simple manner by inserting an electrical plug of a cable that goes to the diathermy generator.

The sample port of the junction slider may be in liquid communication with the second lengthwise channel, which allows resected tissue specimens to be conveyed from the biopsy cup out of the slide rail assembly into the tissue specimen collector for diagnosis. A tissue specimen can simply be flushed out of the sample port by flushing liquid through the first lengthwise extending channel into the closed biopsy cup to push the tissue specimen in fixed orientation into the second lengthwise extending channel. If the first lengthwise extending channel that is used as the delivery channel for the flushing fluid also is used as the third lengthwise extending channel, the electrical wire must be insulated.

The second lengthwise extending channel may have a larger cross-section than the first lengthwise extending channel to inherently further promote ejecting the tissue specimen by flushing. Preferably the second lengthwise extending channel may have the largest cross-section possible in view of the dimensions of the other lengthwise extending channels and the overall diameter of the tube, so that the tissue specimen is treated as gently as possible during its travel out and free of the tube and inside a chamber of the tissue specimen collector, but without the tissue specimen can swirl around inside the second lengthwise extending channel and shift orientation.

Tissue specimens should be deep enough to include both lamina propria and muscularis mucosae, i.e. 3-5 mm in depth. The tissue specimen will automatically be pushed by the pressure of the flushing fluid of the first lengthwise extending channel towards the larger or same cross-section of the second lengthwise extending channel. The tissue specimen is pressed into and along said second lengthwise extending channel by the flushing fluid because the second lengthwise extending channel is the only channel that allows the tissue specimen to slip inside and pass along with the flushing fluid out of the outlet of the sample port. When the tissue specimen is subjected to the pressure of the flushing fluid or a vacuum it may conform slightly to the lumen of the second lengthwise extending channel as regards shape to pass the easiest through said second lengthwise extending channel.

The end effector slider, the junction slider, and/or the needle slider can in an expedient embodiment reciprocate along the guideway of the main slide guide body of the slide rail assembly. In such a configuration of the slide rail assembly the needle or nozzle can extend from the proximal tube end through the junction slider to be connected to the needle slider, whereby the slide rail assembly can be constructed with minimum size. In the alternative the needle or nozzle runs in the third lengthwise extending channel parallel to and outside the junction slider. Within the scope of the present invention the junction slider and the needle slider can be arranged in parallel instead of series, in which case however the proximal end of the needle or nozzle needs to be bend away from the longitudinal axis of the slide rail assembly at its exit from the proximal tube end for being secured to the needle slider. A parallel arrangement of the junction slider and the needle slider makes the slide rail assembly more voluminous, which might obstruct and affect the surgeon's maneuverability of endoscope and endosurgical biopsy device. The third lengthwise extending channel may advantageously be radially offset the longitudinal axis of the tube to arranged the needle to nozzle offset as well, thus no coaxial with the tube and with the working channel of the endoscope such as when using a single needle instrument.

The slide rail assembly may have a first spring member located between the end effector slider and the junction slider for spring-biased reciprocation of the end effector slider against the junction slider. The slide rail assembly may further or additionally have a second spring member located between the junction slider and the needle slider for spring-biased reciprocation of the needle slider against the junction slider. Alternative arrangements that temporarily can keep the sliders under tension or compression in relation to each other are within the scope of the present invention. Such arrangements could be co-operating indents and beads, manually operated reciprocatable pins that e.g. extend crosswise the guideway in front of a distal end of a slider or engaging a slider, etc.

At the storage state and during inserting the endosurgical biopsy device into the working channel of the endoscope none of the spring members may be biased. Once the distal end of the endoscope is in position at the suspicious-looking tissue target and the endosurgical biopsy device and the patient is ready for biopsy, the endosurgical biopsy device can be prepared for resecting the tissue specimen by inserting the tube in the working channel such that a tissue specimen optically detected using the fiber optics of the endoscope can be resected. The junction slider is operated to move the end effector out of the endoscope. Then the end effector slider is operated to retract the effector sleeve thereby allowing opposite jaws of the end effector to get apart to grasp a tissue specimen and apply diathermy. To expose the distal tube end with the end effector and effector sleeve from the endoscope's distal opening, the junction slider travels in the guideway without compressing the spring members. If anesthetics are needed at the biopsy site the needle slider is then moved forward towards the junction slider thereby compressing the second spring member between the needle slider and the junction slider to expose the needle or nozzle in relation to an end effector which was opened by operating the end effector slider.

Spring-biasing the needle slider by compression of the second spring member reduces the risk that the end effector is retracted inside the effector sleeve while the needle or nozzle is still in its forward exposed position.

The compression of at least the second spring member may also contribute to that the jaws do not break inside the organ by accident by accidentally closing around an exposed needle. The needle will automatically retract when compression force is relieved.

As a further safety precaution the spring members can advantageously be compression springs, such as coils, having different numbers of coils per unit length, and optionally also one or more different parameters selected from different coil diameter and different wire thickness, in order to be loaded to a different compression, e.g. so that the second spring member springs back faster than the first spring member upon release of compression and load on said spring members. As already mentioned above this arrangement of spring members provides safety against the end effector accidentally and unintentionally closes prior to the needle or nozzle has been pulled inside the biopsy cup again. A suitable structural and functional precaution to prevent the end effector from catching the exposed needle or nozzle can thus simply be facilitated by configuring the second spring member with a larger compression force than the first spring member whereby the second spring member retracts the needle slider faster than the first spring member is relieved of the compression force between the end effector slider and the junction slider.

The sliders of the slide rail assembly can be operated manually and hold on to by the surgeon. This may however be a somewhat clumsy way to operate the slide rail assembly, and not safe and reliable. A safer and more practical way to operate the end effector via the slide rail assembly can be a remote actuator assembly having an remote operating handle with buttons allocated to operating the respective sliders and functionalities, and being operatively connected to the sliders via respective operating strings. The operating strings can e.g. be wires, such as metal wires, but any kind of string having same strength and similar ability to bend and flex without rupturing can be used within the scope of the present invention. Strong polymeric strings, such as e.g. fiber reinforced strings, is an example of an alternative to metal wires.

The remote actuator assembly may comprise a sheave assembly, which sheave assembly can be located at the junction slider to move together with said junction slider in response to pulling an operating string by operating a respective button of the remote operating handle. The operating string may loop around the sheaves of the sheave assembly one or more times. The more times the larger mechanical advantage can be obtained. A small stroke of a button can thus cause a longer displacement of a slider.

A further advantageous feature of the remote operating handle is that the remote operating handle can have a snap-on means for displaceable and detachable attachment of the remote operating handle to the distal part of the endoscope, such as the flexible tube part of the endoscope, whereby said remote operating handle is easy at hand of the surgeon at all times during the biopsy procedure. The remote operating handle can be snapped-on/snapped off the distal tube part of the endoscope both in parallel or perpendicular as the surgeon or operator prefers, and slide along said distal tube part.

If the surgeon prefers so he/she can detach the remote operating handle from the distal part of the endoscope to operate the slide rail assembly in any of his/her preferred positions. He/she can also slide the remote operating handle lengthwise along the distal part, or temporarily store the remote operating handle when not in use so that it is not in the way at the proximal end of the endoscope. The remote operating handle of the present invention gives the surgeon more ergonomic operating conditions and eliminates the need for some of the additional staff needed during a conventional endoscopic biopsy procedure.

Prior to resecting the tissue specimen the patient is prepared for the biopsy. Such preparation can sometimes include flushing an irrigant, such as a saline solution, through the endoscope to rinse or optionally expand the organ or body cavity. If the organ is the urinary bladder a cystoscope may be used and the urinary bladder be inflated with the saline solution to rinse the urinary bladder to a level that gives a clear vision of the urinary bladder wall. The organ or body cavity may typically be emptied of any electrically conductive flushing/rinsing solution prior to tissue acquisition by diathermy. The flushing/rinsing solution can be collected for histology analysis and diagnosis of pathologies.

Saline solutions are the typical irrigant solution. Saline solutions are however conductive and therefore not compatible with diathermy. So prior to biopsy the saline solution is replaced with a non-conductive flushing solution, e.g. a glycine solution, a solution of other non-conductive amino acids, or a sugar solution such as e.g. a mannitol solution. A glycine solution, of e.g. 1.5%, can be used as flushing solution instead of saline during diathermy, and for distending the organ in preparation for biopsy.

The device operating part may further comprise a flushing component having a first operating end part adapted to be mounted to an endoscope fluid valve port and to an endoscope suction valve port of an endoscope, and an opposite second operating end part adapted to couple to the slide rail assembly and to the endoscope at or in the vicinity of the instrument port of the endoscope.

Initially the organ or tissue site may be rinsed using a saline solution and the conventional procedure and suction/flushing channel of the endoscope.

Alternatively the flushing component may initially be used for the same purpose as a shunt bypassing the proximal parts of the suction channel and the liquid channel of the endoscope between the fluid valve port and the suction valve port and the instrument port, to make liquid flow via the instrument port instead and out of the distal end of working channel of the endoscope thereby flushing the organ or tissue site of interest via the working channel and the tube, and evacuate infused liquid again in a later step via the tube, thereby rinsing the organ prior to biopsying and being able to collect evacuated flushing solution.

The opposite second operating end part may conveniently be adapted to be coupled to the instrument port of the endoscope device operating part via an adaptor coupled to the instrument port. In the alternative the adaptor is an integral part of the second operating end part.

The adaptor may include a tubular adaptor body that has a first adaptor end part with a first adaptor end, and an opposite second adaptor end part with a second adaptor end. The first adaptor end part may be dimensioned to replace the end cap of the instrument port, preferably including a membrane for introduction of the tube. The second adaptor end part may be configured for coupling with the slide rail assembly to hold the slide rail assembly together with the endoscope at the instrument port and keep control of any tubing leading to and from the slide rail assembly and supplies and discharges of liquid.

The first adaptor end part and the second adaptor end part may be at an angle in relation to each other, so that the slide rail assembly is directed at least slightly away from the longitudinal axis of the endoscope and thereby being highly accessible and operational by the surgeon. The first adaptor end part may conveniently be configured similarly as the instrument port of the endoscope with a membrane through which the tube of the endosurgical biopsy device of the present invention can be inserted into the working channel of the endoscope, enabling liquid to be supplied into the first lengthwise extending channel and leave via the second lengthwise extending channel.

Preferably the adaptor can also rotate in the instrument port, such as at least 45°, about a central axis of the endoscope's instrument port, to further provide improved operating conditions for the surgeon or other operator. In the alternative the slide rail assembly can rotate in relation to the adaptor in a similar manner about a central axis of the endoscope's instrument port, to thereby also rotate the end effector.

Establishing fluid communication between one or more of liquid reservoirs, containers for collecting discharged matter, the first and second lengthwise extending channel of the tube, and the junction slider of the slide rail assembly requires tubes there-between. It may thus be expedient if the adaptor has seats for coupling pieces for mounting one or more tubes to one or more of the components selected from the group comprising at least one or more pump means, a liquid reservoir, a drainage reservoir, the inlet port of the junction slider, the sample port of the junction slider, a collector port to a tissue specimen collector secured to the sample port or an outlet from the tissue specimen collector secured to the sample port, to obtain liquid communication and flow channels between respective such parts.

The opposite jaws of an end effector of the forceps kind may be obtained from two lengthwise pipes sections subsequently joined partly lengthwise and having jaws deflected from the lengthwise longitudinal axis of the end effector to confer an inherent resiliency to said jaws that will make the jaws to spring apart when the effector sleeve does not keep the jaws closed. Any of the end effectors described in the applicant's Swedish patent application no. 1751639-4 filed 22 December 2017 and the applicants international patent application PCT/SE2018/051315 14. December 2018 can be part of the endosurgical device of the present invention.

The biopsy cup can be closed sealingly by the reciprocating movement of the effector sleeve induced by the reciprocating movement of the end effector slider to prevent flushing fluid from escaping the closed biopsy cup, and thus loosing flushing pressure, thus to substantially prevent flushing fluid from flowing into the organ or out to the biopsy site via crevices and/or gaps between opposite closed jaws when a tissue specimen is flushed out of the closed biopsy cup.

The exterior diameter of the tube may be as little as less than or equal to 2 mm for the endosurgical biopsy device to fit moveably inside a working channel of a cystoscope once a heat-shrink insulated reinforcing coil is put around the tube. However other kinds of endoscopes may have larger working channels, in which case the overall exterior diameter of the tube with enclosures can be as wide as about 2.5 mm, 3 mm, 3.5 mm, 4 mm, 4.5 mm, or even about 5 mm or above. The interior diameter of any of the lengthwise extending channels is smaller than the overall interior tube diameter, as measured as the largest distance between the lumens of the lengthwise extending channels.

The flushing pressure of the flushing liquid or the vacuum may be set in view of the diameter of the lengthwise extending first and second lengthwise extending channels of the tube and the size of the tissue specimens, as defined by the jaws of the end effector and of the closed biopsy cup, so that the tissue specimens are not trapped inside the second lengthwise channel, thereby avoiding and/or preventing plugging of said second lengthwise channel. For an endosurgical biopsy device that implements a tube of an exterior overall diameter of 2 mm for all functionalities, and being intended for use with a cystoscope, a rather high flushing fluid delivery pressure through the second lengthwise channel of at least 5 bar could be needed. The fluid delivery pressure may however be as high as 10 bars, or 20 bars, or even higher. A fluid delivery pressure of e.g. 26 bars has been demonstrated to be satisfactory and efficient.

The free opposite edges of cup-shaped second ends of the jaws form conductive pinching surface for contacting the tissue in application of diathermy when the jaws are moved towards each other, e.g. by pulling at an effector wire or moving the effector sleeve so that the end effector is closed and arranged inside the effector sleeve.

Diathermy remedies the need for the surgeon to pull at the end effector extensively to mechanically resect the tissue specimen, and the need for hemostasis by heat application subsequent to resection of the tissue specimen is highly reduced or even eliminated. The surgeon only has to wait a few seconds for the applied heat to set the tissue specimen free at very little discomfort to the patient and with minimum steps for the surgeon. Once the tissue specimen is set free, a force applied to the effector sleeve can make the biopsy cup to close fully. Alternatively the jaws can be proactively closed by means of the effector wire coupled to the end effector slider. Once the end effector has been closed the flushing liquid can pass through it without the biopsy cup leaks noticeably to loose flushing pressure. The tissue specimen is thereby flushed out of the second lengthwise extending channel and collected in a dedicated chamber of the tissue specimen collector. Once all tissue specimens have been collected by the tissue specimen collector, all the tissue specimen can be brought to the pathologist in a closed environment in a single cassette or cartridge as constituted by the tissue specimen collector. No human contaminating interaction is needed to move the tissue specimens out of the organ and collect it into vials, such as a conventional vial in a conventional biopsy. Contamination risk is zero during surgery from tissue specimen acquisition to tissue specimen collection, and mixing up tissue specimens is eliminated.

The jaws pinch the tissue specimen and in the diathermy step the jaws gently sever the tissue specimen from the organ, and sear and/or cauterize the wound left at the biopsy site to stop bleeding. In this manner the tissue specimen releases from the organ and do not stick to the jaws.

The exterior faces of the opposite jaws may be electrically insulated exteriorly to constitute a diathermy device that allows the tissue specimens to release from the organ. Electric insulation of the exterior faces of the jaws may preferably be obtained by providing a coating to said exterior surfaces. Such coating may preferably be a low friction coating, e.g. a Parylene^{®} coating or a coating of Diamond-like carbon (DLC) that easily can be deposited on a conductive surface of the jaws, such as a metal surface, the metal being e.g. nitinol, aluminium or stainless steel. The trade name Parylene^{®} covers chemical vapor deposited poly(p-xylylene) polymers often used as moisture and dielectric barriers. Within the scope of the present invention other kinds of insulating coatings can also be used, e.g. Diamond-like carbon (DLC).

The tube may be a non-conductive tube, such as a plastic tube. If excess bleeding occurs diathermy can be repeated locally. The diathermy may be monopolar.

Preferably the organ is the urinary bladder, but other organs can be subjected to same principles and biopsy procedure, e.g. can the organ be the digestive tract, such as the intestine, stomach or esophagus; or the airways, such as the lung; or the vagina.

The needle or nozzle associated with the third lengthwise extending channel may be utilized for other purposes than anesthesia, such as for delivering expanding fluid to an organ or tissue site, inject medication, such as local anesthesia or adrenaline to stop bleeding. Other options include but are not limited to using a surgical laser or a clamp tool through the fourth lengthwise extending channel.

The steps of the surgical procedure using the endosurgical biopsy device of the present invention for taking tissues specimens may comprise the steps of
a) inserting an endoscope, preferably an endoscope having means to visualize the organ internally, such as a fiber optic means,
b) optionally expanding the organ wherefrom tissue specimens are to be resected with liquid supplied through the working channel or other channel of the endoscope,
c) inserting a tube of the endosurgical biopsy device and, optionally if not performed in step b), expanding the organ wherefrom tissue specimens are to be resected with non-conductive liquid supplied through a lengthwise extending channel of the endosurgical biopsy device,
d) optionally anesthetizing all areas suspect of cancer, or optionally anesthetizing locally at the sampling site, by operating the needle or nozzle of the endosurgical biopsy device via the needle slider, optionally inducing swelling to facilitate taking the tissue specimen,
e) by operating the junction slider exposing from the working channel of the endoscope the distal end of the tube of the endosurgical biopsy device, in a state where the effector sleeve surrounds the end effector,
f) displacing the end effector and the effectors sleeve in relation to each other by operating the end effector slider, thereby opening and closing the jaws of the end effector to pinch a tissue specimen,
g) activating diathermy via the diathermy wire of the junction slider to provide current to the end effector to set the tissue specimen free for accommodation inside the biopsy cup,
h) from the junction slider flushing non-conductive liquid under high pressure into the first lengthwise extending channel and further into the closed biopsy cup of the end effector to flush the tissue specimen out of the tube via the second lengthwise extending channel of the tube, which second lengthwise extending channel ends at the junction slider, and collecting the tissue specimen at the proximal tube in a tissue specimen collector as defined in any of the preceding claims,
i) repeating steps e) - h) in case of anesthetizing all areas suspect of cancer has been anesthetized, or in case of local anesthesia repeating steps d) - h) until the relevant number of tissue specimens has been taken,
j) optionally destroying any remaining areas of cancer by burning function by moving the jaws to the tissue being destroyed,
k) withdrawing the endosurgical biopsy device, and
l) withdrawing the endoscope.

Step j) can be conducted at any stage of the biopsy procedure, and in fact since the same endosurgical biopsy device is used during one inserting of the endosurgical device in the working channel of the endoscope any desired steps can be conducted and repeated in accordance with the surgeons choice without removing the endosurgical biopsy device from the working channel. Thus the order of the steps indicated in the surgical procedure should not be construed as limiting and mandatory.

The invention will now be described below with reference to the drawing by way of an exemplary embodiment of an endosurgical biopsy device of the present invention.
Fig. 1 is a perspective overview of an endosurgical biopsy device of the present invention in its operative set up environment ready for use in a biopsy procedure using a cystoscope,
fig. 2 is an enlarged cross-sectional view taken along line II-II in fig. 1 of a tube for the endosurgical biopsy device of the present invention,
fig. 3 is fragmentary perspective end view of an embodiment of an end effector,
fig. 4 a perspective, enlarged scale view of the endosurgical biopsy device seen in fig. 1 mounted at a proximal handle part of a cystoscope, but with the electrical cord to the diathermy generator left out and liquid supply tubes and discharge tubes also left out,
fig. 5 shows, in perspective and in partial exploded view, the slide rail assembly, the first embodiment of a tissue specimen collector and the adaptor,
fig. 6 shows the same in assembled state,
**fig.** 7 is an enlarged scale view of the adaptor of the device operating part seen from the side,
fig. 8 shows the flushing component from the holder end and the first operating end part,
**fig.** 9 shows the same from the second operating end part,
figs. 10 and 11 are exploded, enlarged scale views of the endosurgical biopsy device seen from the distal guide end and the proximal guide end, respectively, of the main slide guide body without the spring members, the adaptor and the flushing component,
fig. 12 is a perspective enlarged scale fragmentary view of the encircled fragment to the right in figs. 15 and 16 of the endosurgical biopsy device seen from the distal tube end and with remote actuator assembly,
**fig.** 13 shows the remote operating handle in perspective view from above,
**fig.** 14 shows the same with a casing-half left out,
fig. 15 shows the endosurgical biopsy device in its starting position,
fig. 16 shows the endosurgical biopsy device in its configuration where the operating end of the endosurgical biopsy device has been exposed from the endoscope's working channel,
fig. 17 shows the endosurgical biopsy device with the effector sleeve retracted from the end effector,
fig. 18 shows the encircled operating end of the endosurgical device seen in fig. 17 in an enlarged scale view,
fig. 19 shows the operating end of the endosurgical device with the needle exposed between the jaws of the end effector,
fig. 20 shows the encircled operating end of the endosurgical device seen in fig. 19 in an enlarged scale view,
fig. 21 is a perspective view of a second embodiment of a tissue specimen collector seen from its distal end,
fig. 22 shows the same from its proximal end,
fig. 23 is an exploded perspective view of the second embodiment of a tissue specimen collector seen in fig. 21,
fig. 24 shows the second embodiment of a tissue specimen collector from the side,
fig. 25 is an exploded view of the second embodiment of the collector rack assembly seen in fig. 23.
fig. 26 shows the collector rack assembly seen in fig. 24 inclined from the side opposite the linear rack,
fig. 27 shows the collector rack assembly seen in fig. 24 inclined from above,
fig. 28 shows the collector rack assembly seen in fig. 24 inclined from the linear rack side,
fig. 29 shows the push rod part from the pawl tip,
fig. 30 is a sectional view taken along line XXX-XXX in fig. 23 inside the proximal tubular housing part, and
fig. 31 is a sectional view taken along line XXXI-XXXI in fig. 23 inside the distal tubular housing part.

In the following it is by way of example assumed that the endoscope is a cystoscope and the organ, wherefrom tissue specimens are to be resected, is the urinary bladder. The examples are non-limiting and it should be understood that the endosurgical biopsy device of the present invention and the tissue specimen collector can be used with many other kinds of endoscopes, and that the target tissue can be many different kinds of tissue. It is emphasized that scaling used in the figures are made to illustrate features of the invention the best possible way. Ratio of scaling up and down may differ between different figures. Scaling is chosen arbitrarily to illustrate components of the invention the best. Other end effectors, such as scissors and other forceps designs, can be used in the endosurgical biopsy device.

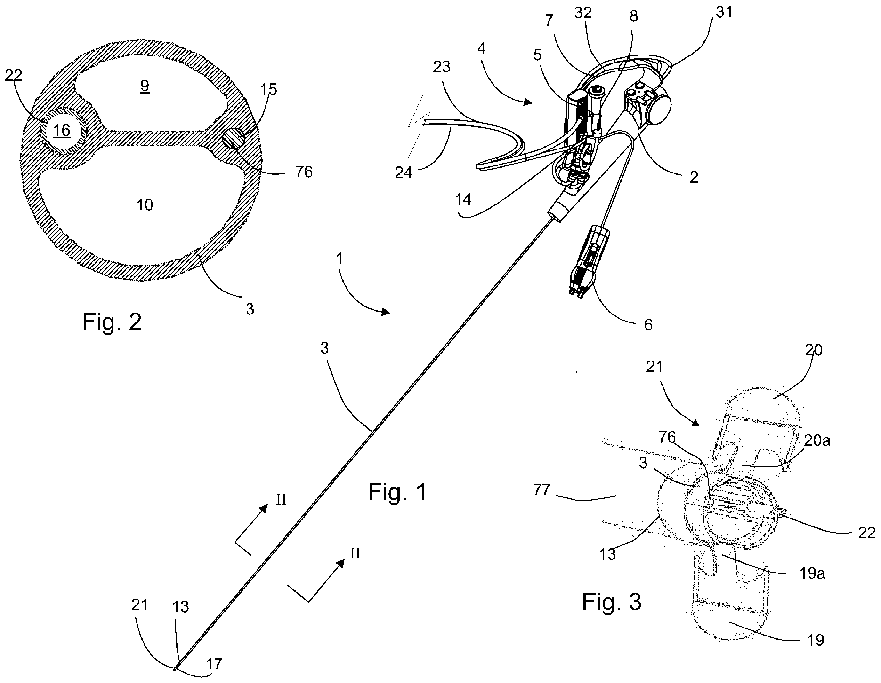

Fig. 1 is a perspective overview of an endosurgical biopsy device 1 of the present invention in its operative set up environment ready for use in a biopsy procedure with an endoscope in form of a cystoscope 2. A plurality of conventional diathermy generators, liquid sources, and reservoirs can be used in the biopsy procedure. Those are not shown in fig. 1. Furthermore, for the purposes of visual overview the distal insertion tube of the cystoscope and the fiber optic have been left out. The multi-channel tube 3 is shown uncovered by the distal insertion part of the endoscope.

The endosurgical biopsy device 1 has a device operating part 4, which comprises plural sub-components: including at least a slide rail assembly 5, a remote actuator assembly 6, a flushing component 7, and a first embodiment of a tissue specimen collector 8. Each of these sub-components 5,6,7,8 will be described separately and in combination in further details in the following figures, as well at their operative relationship will be explained and discussed.

The flexible multi-channel tube 3, in the following just referred to as the tube, used in the endosurgical biopsy device 1 seen in fig. 1, is shown in enlarged cross-sectional view in fig. 2.

The tube 3 has four lengthwise extending channels: a first lengthwise extending channel 9 for supplying liquid to the distal tube end 13 of the tube 3, a second lengthwise extending channel 10 for removing, e.g. by expelling or aspirating, liquid and other matter from the tube 3, such as tissue specimens, out via the proximal tube end 14, a third lengthwise extending channel 15 for a diathermy wire 76, and a fourth lengthwise extending channel 16 for reciprocating a needle. It could be said that the lengthwise extending channels "divide" the working channel of the endoscope into several sub-channels that improve the functionality of the endoscope to a hitherto unknown extent and dispense with the need to insert and retract different instruments through the working channel of the endoscope numerous times, thus saves time for biopsying, and confers comfort to the patient.

An embodiment of an end effector 21 is seen in fig. 3 in an enlarged scale view. The effector sleeve 17 seen in fig. 3 has an internal diameter selected for the end effector 21 to move tightly in and out of said effector sleeve 17, e.g. in response to reciprocating the end effector 17 at the distal tube end 14 of the tube 3 in and out of the effector sleeve 17, or by reciprocating the effector sleeve 17 over and away from the end effector 21, thus reciprocating the end effector and the effector sleeve in relation to each other once the effector sleeve surrounded end effector 21 is placed in its biopsy position in front of the target site. When the end effector is in this biopsy position at the target site the jaws 19,20 are opened by retracting the effector sleeve. Next the tissue specimen is grasped by closing the jaws 19,20 by displacing the effector sleeve in the opposite direction above the end effector to make flexible hinge members 19a,20a of respective jaws to flex back. The effector sleeve 17 may be a metal pipe and the tube 3 may have an exterior structural reinforcement, such as a coil member 77, to provide the tube 3 with sufficient structure to be inserted into the working channel of the endoscope, and optionally to allow lengthwise movement in relation to the effector sleeve 17. In fig. 3 of the end effector 17, the needle 22 has been exposed from the third lengthwise extending channel 16. The exterior structural reinforcement may be secured end-to-end to the effector sleeve and reciprocate simultaneously.

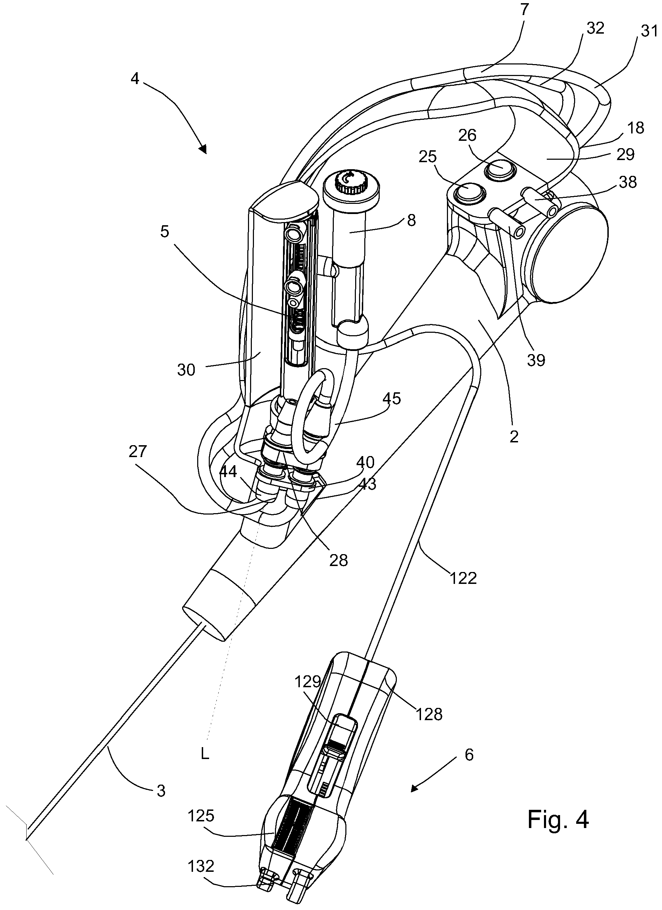

Fig. 4 is an enlarged scale perspective view of the endosurgical biopsy device 1 seen in fig. 1, but without the electrical wire that connects the endosurgical biopsy device 1 to the diathermy generator, and without the flushing and evacuation tubes 23,24 to a priming pump (not shown) and without liquid reservoirs and liquid sources. A priming pump may be associated with the diathermy generator and adapted to fill the tube system coupled to the tube 3, including flushing via the slide rail assembly 5 the first lengthwise extending channel 9 with non-conductive liquid and collect aspirated/discharged non-conductive liquid in connection with expelling tissue specimens from the second lengthwise extending channel 10.

The flushing component 7 has a first operating end part 18 adapted to be mounted to an endoscope fluid valve port by means of a first push-activated valve 25 and to an endoscope suction valve port of the cystoscope 2 using a second push-activated valve 26. An opposite second operating end part 27 of the flushing component 7 is in fluid communication with the slide rail assembly 5 and with the instrument port of the cystoscope 2 by means of an adaptor 28 at said second operating end part 27.

The second push-activated valve 26 opens and closes for the supply of liquid from a source of liquid (not shown), e.g. a drip bag, coupled to an infusion connection piece 38 to infuse liquid through the cystoscope via the infusion tube 31 of the flushing component 7. Similarly the first push-activated valve 25 opens and closes for suction through the endoscope via the suction tube 32, which suction tube 32 is coupled to a vacuum source via a suction connection piece 39.

The grip 29 makes it easy for the surgeon to hold the endosurgical biopsy device and the cystoscope at the same time by simply placing the hand on the cystoscope through the grip 29. The grip 29 may incorporate internal irrigation tubes or have external irrigation tubes 31,32 for flushing liquid, such as saline, water or glycine, and evacuating liquid again, respectively, from the hollow organ, such as the urinary bladder, or other tissue surface, through the working channel of the cystoscope 2 via the tube 3, or through a liquid channel (not shown) of the cystoscope 2 especially allocated to said purpose prior to conducting biopsy.

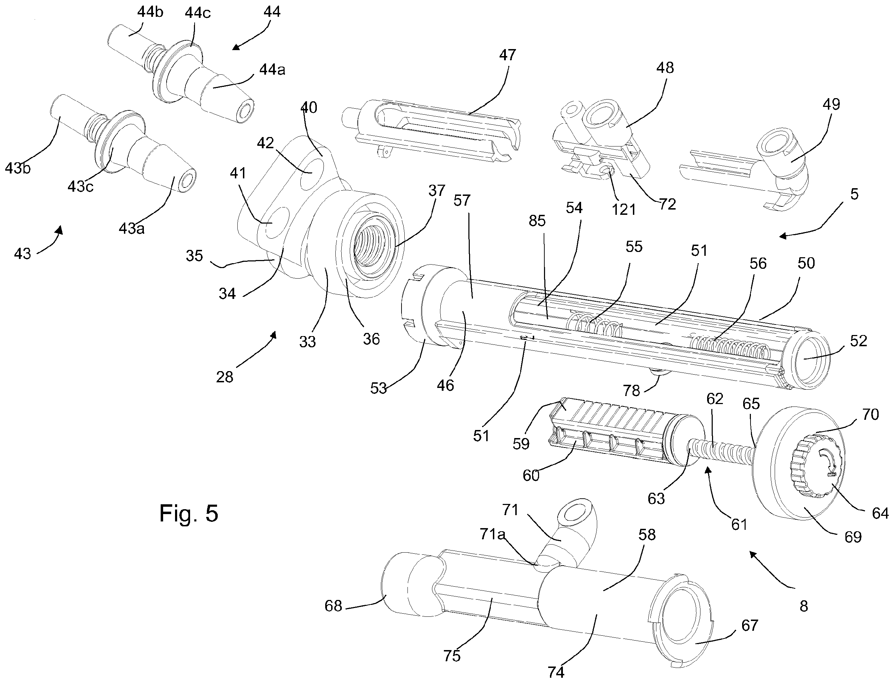

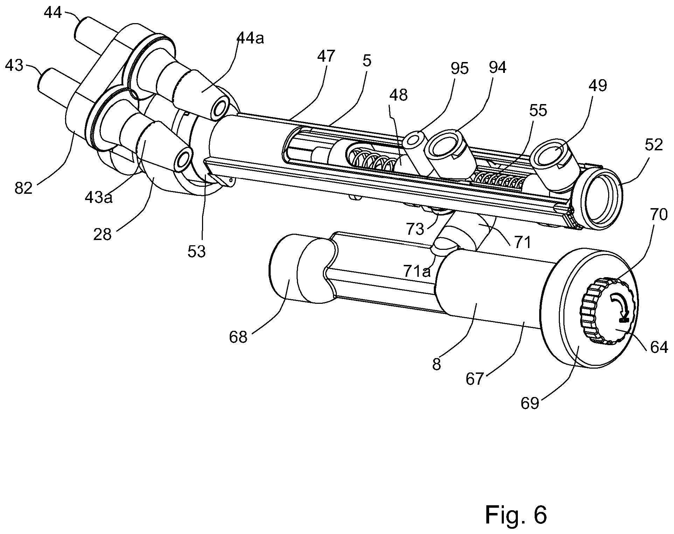

The perspective, exploded view of fig. 5 and the assembled view of fig. 6 show the slide rail assembly 5, the first embodiment of a tissue specimen collector 8 and the adaptor 28. Said adaptor 28 has a tubular adaptor body 33 that has a first adaptor end part 34 with a first adaptor end 35, which first adaptor end part 34 has a membrane (not shown) for inserting the tube 3, and an opposite second adaptor end part 36 with a second adaptor end 37 configured for coupling with the slide rail assembly 5.

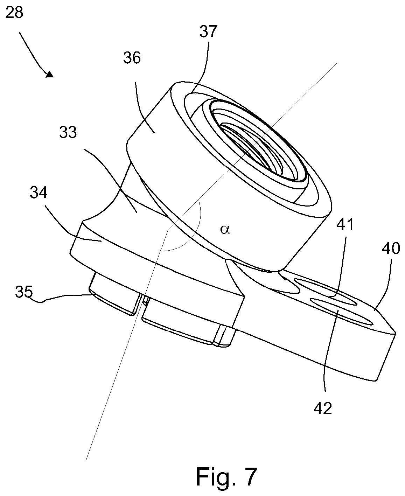

The first adaptor end part 34 and the second adaptor end part 36 are, as seen best in the side view of fig. 7, at an angle α in relation to each other, so that the slide rail assembly 5, which is secured to the second adaptor end part 36 is turned away from the cystoscope 2. The angle α is e.g. at least 45° to define a bend or curved flow channel through the tubular adaptor body 33.

The irrigation tubes 31,32 associated with the grip 29 are the infusion tube 31 and the suction tube 32. The suction tube 32 take part in application of suction to empty the urinary bladder, and the infusion tube 31 take part in infusing liquid to e.g. rinse the urinary bladder prior to the biopsy procedure and instill non-conductive liquid into the urinary bladder.

The adaptor 28 has a protruding flange 40 with seats 41,42. The seats 41,42 are adapted for mounting of a first coupling piece 43 and a second coupling piece 44 for providing fluid communication to the suction tube 32 and the infusion tube 31, and to the vacuum source (not shown) and the liquid source (not shown), respectively. The first and second coupling pieces 43,44 may e.g. be barbed bayonet male connectors, having respective barbed tapering first coupling ends 43a,44a and opposite second coupling ends 43b,44b with an enlarged diameter flange 43c,44c in-between said coupling ends 43a,44a;43b,44b to promote the coupling pieces 43,44 staying in their respective seats 41,42 at the protruding flange 40.

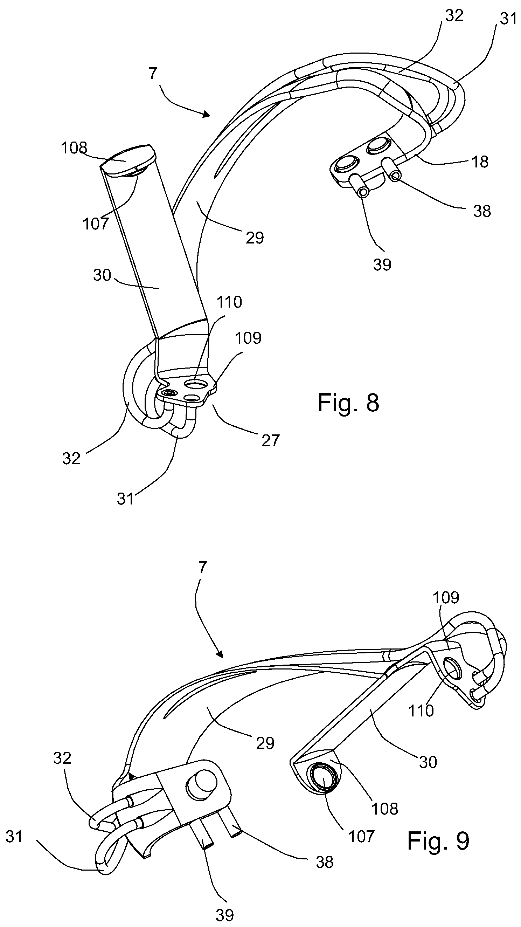

As seen best in the enlarged scale detailed views of figs. 8 and 9 the flushing component 7 has a longitudinal grip 29 that extends between the first operating end part 18 and the second operating end part 27 of said flushing component 7. At the second operating end part 27, the flushing component 7 has a holder 30 for the slide rail assembly 5. The slide rail assembly 5 that is coupled to the first embodiment of a tissue specimen collector 8 is docked in the holder 30 to be kept in a position protruding from the cystoscope 2. Both the slide rail assembly 5 and the first embodiment of a tissue specimen collector 8 are thus located close to the proximal end of the cystoscope 2, and in convenient reach and view of the surgeon.

In use of the endosurgical biopsy device 1 the adaptor 28 is secured in the instrument port, the second operating end part 27 of the flushing component 7 is secured to the adaptor 28, the slide rail assembly 5, which is connected to the first embodiment of a tissue specimen collector assembly 8, is mounted in the holder 30, and the tube 3 with needle 22, end effector 21, effector sleeve 17, and diathermy wire 76 is inserted through the membrane of the adaptor **28.** The suction tube 32 is connected to the barbed tapering first coupling end 43a of the first coupling piece 43. The infusion tube 31 is connected to the second coupling end 44b of the second coupling piece 44. An intermediate tube 45 connects the first embodiment of a tissue specimen collector 8 to the barbed coupling end 43a of the first coupling piece 43 to establish liquid communication to the suction tube 32 of the grip 29 to evacuate liquid from the first embodiment of a tissue specimen collector 8.

The flushing tube 24 is connected to the tube coupling end 44a of the second coupling piece 44 to establish liquid communication to the primping pump (not shown). The opposite tube coupling end 44b of the second coupling piece 44 connects to a source of non-conductive liquid via the infusion tube 31 of the grip 29 thereby establishing liquid communication between priming pump (not shown) and source of non-conductive liquid (not shown). Non-conductive liquid can thus be consecutively replenished from same or another source coupled to the infusion tube 32 via the flushing component 7 after the closed biopsy cup has been evacuated by application of suction to the first embodiment of a tissue specimen collector 8 and the priming pump (not shown) is to be refilled for the next resecting of a tissue specimen.

The slide rail assembly 5 has, as shown in figs. 5 and 6, a main slide guide body 46 for slidingly suspending an end effector slider 47, a junction slider 48, and a needle slider 49. The main slide guide body 46 is comprised of a tubular guide housing 50 with interior, opposite, lengthwise extending interior tracks 51. The opposite interior tracks 51 allow the end effector slider 47, the junction slider 48, and the needle slider 49 to reciprocate more or less to and from a proximal guide end 52 and an opposite distal guide end 53 of said main slide guide body 46. The main slide guide body 46 with the interior tracks 51 then defines a guideway 54 for the sliders 47,48,49 to reciprocate along.

A first spring member 55 is inserted in the tubular guide housing 50 between the end effector slider 47 and the junction slider 48, and a second spring member 56 is inserted between the junction slider 48 and the needle slider 49 to spring-bias any of said sliders 47,48,49 when they slide along the length of the guideway **54.** In addition to the interior tracks 51 the wall 57 of the main slide guide body 46 also has a lengthwise extending opening 85, which is shorter than the interior tracks 51 to define the maximum possible stroke distance of the junction slider 48 and of the needle slider 49 in view of also the length of the end effector slider 47, but still allowing the end effector slider 47 to slide close to the distal guide end 53 of the main slide guide body 46.

The end effector slider 47 is connected to the effector sleeve, via a reinforcing coil 77, and by moving the reinforcing coil 77 in relation to the tube 3 the effector sleeve 17 moves lengthwise in relation to the end effector 17 and the jaws 19,20 open and/or close.

The junction slider 48 is connected to the proximal end 14 of the tube 3 and is in fluid communication with the first lengthwise extending channel 9 and the second lengthwise extending channel 10 of the tube 3. The junction slider 48 also provides electrical communication between the end effector 21 and a diathermy generator (not shown) by means of an electrical wire 76 through the third lengthwise extending channel 15 of the tube 3. The electrical wire 76 is kept insulated from liquid flowing inside the first and second lengthwise extending channels 9,10 by the surrounding tube's 3 wall. The electrical wire 76 is externalized via the junction slider 48 to be brought in electrical contact with the diathermy generator (not shown) to apply current to the end effector 21 to perform diathermy.

The structure of the end effector slider 47, the junction slider 48, and the needle slider 49 will be described in further details below.

The first embodiment of a tissue specimen collector 8, which is seen in partly exploded view in figs. 5 and 6, has a main collector housing 58, a collector rack 59 with a series of adjacent chambers 60 for accommodating tissue specimens, and means 61 for moving the collector rack 59 lengthwise along the longitudinal axis of the main collector housing 58, which means 61 in the present exemplary embodiment is a screw rod 62 that engages with a threaded hole 63 or bore in the collector rack 59. The screw rod has a rotating knob 64 at a free end 65 facing away from the collector rack 59 and an opposite end 66 that threadingly engages the collector rack 59 to displace said collector rack 59 in response to rotating the rotating knob 64.

The main collector housing 58 is tubular and has a proximal housing end 67 that is threaded to mount a detachable cap 69, and an opposite liquid outlet 68 to be coupled to the intermediate tube 45, as seen in e.g. fig. 4, to discharge flushing liquid entering the first embodiment of a tissue specimen collector 8 from the junction slider 48 of the slide rail assembly 5, when the second lengthwise extending channel 10 is being flushed or evacuated to carry a tissue specimen out of the tube 3.

Other kinds of coupling means between the main collector housing 58 and the detachable cap 69, such as male and female snap couplings are also suitable. The detachable cap 69 facilitates that the collector rack 59 with the tissue specimens can be taken out of the main collector housing 58 after completing the biopsy procedure. The free end 65 of the screw rod 62 passes through a cap hole 70 in the detachable cap 69 so that the rotating knob 64 is accessible from outside the main collector housing 58. Preferably, the cap hole 70 is at the centre of the detachable cap 69 to allow the screw rod 62 to rotate, which cap hole 70 thus also serves to keep the screw rod 62 substantially centered during the surgery so that the position of the chambers 60 is easily shifted and the collector rack 59 does not skew or gets misaligned during its translatory movement in the main collector housing 58.

The main collector housing 58 has a collector port 71a, which, via a protruding coupling piece 71, fluidly couples the tissue specimen collector 8 to a sample port 72 of the junction slider 48, which sample port 72 protrudes from the lengthwise extending opening 85 of the tubular guide housing 50 of the main slide guide body 46.

The main collector housing 58 has at least two lengthwise extending sections: a proximal tubular housing end part 74 and a distal tubular housing end part 75 in extension thereof. The proximal tubular housing end part 74 accommodates the screw rod 62, and depending on which chamber 60 of the series of chambers of the collector rack 59 that is brought into communication with the collector port 71a and the sample port 72 via the coupling piece 71, the proximal tubular housing end part 74 also accommodates more or less of the length of said collector rack 59. The proximal tubular housing end part 74 ends in the proximal housing end 67. Opposite said proximal housing end 67 the proximal tubular housing end part 74 extends into the distal tubular housing end part 75 that, depending on which chamber 60 of the series of chambers of the collector rack 59 that is brought into communication with the collector port 71a and the sample port 72 via the coupling piece 71, accommodates the entire collector rack 59 or a part of the collector rack 59 only. The cross-section of the distal tubular housing end part 75 may corresponds substantially to the cross-section of the collector rack 59, to avoid that the collector rack 59 rotates inside the distal tubular housing end part 75 about its longitudinal axis so that a chamber 60 cannot be aligned with the sample port 72 of the junction slider 48.

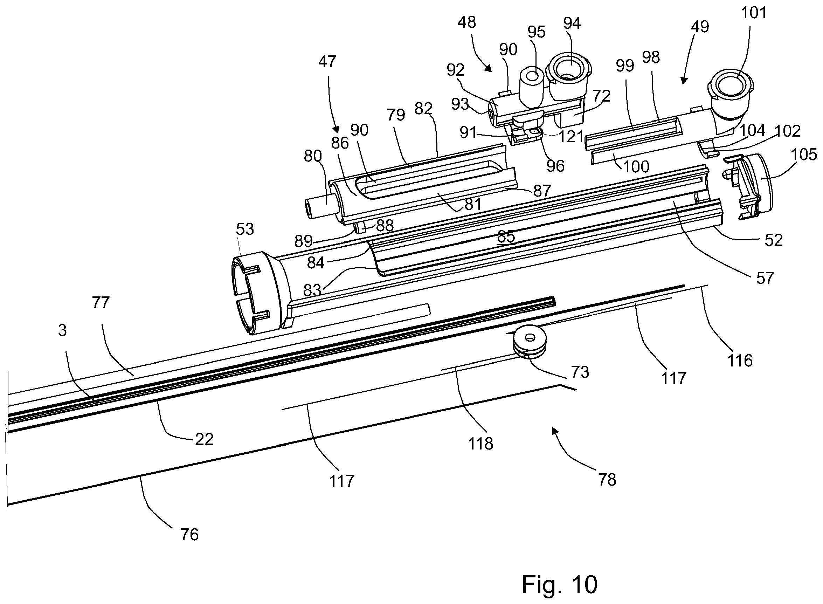

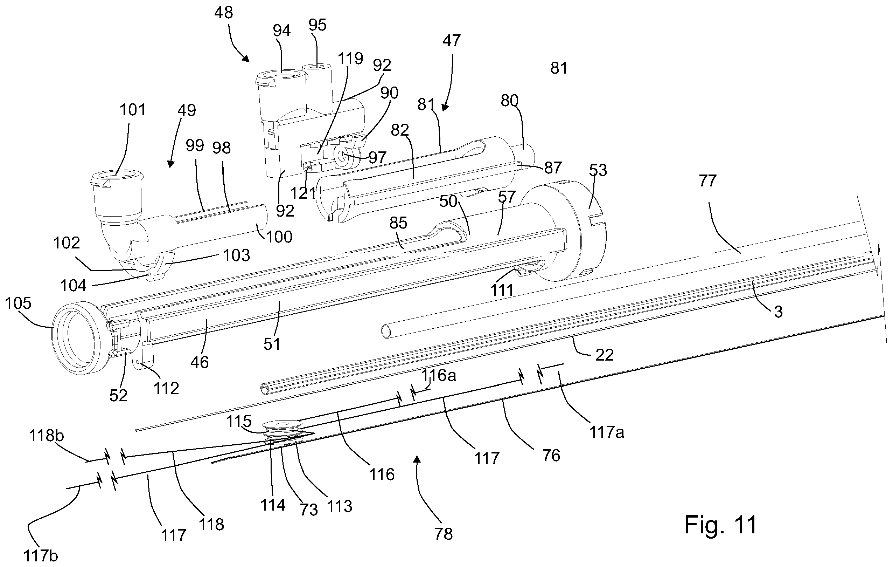

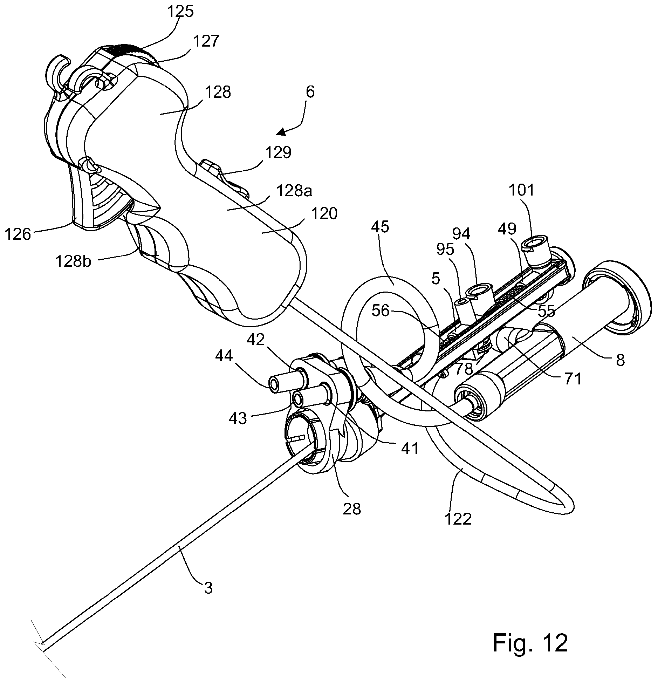

The sliders 47,48,49 are shown in perspective in figs. 10 and 11, and seen assembled into the slide rail assembly 5 in fig. 12. Figs. 10 and 11 also show, in perspective, the tube 3 and the reinforcing coil member 77 for the tube 3, which reinforcing coil member 77 may be secured distally to the effector sleeve 17, e.g. by means of an exterior heat-shrinking tube, the needle 22. Figs. 10 and 11 further show the diathermy wire 76, and the sheave assembly 78 from different ends. The spring members 55,56 are left out to better overview the structural features of the sliders 47,48,49, and of the main slide guide body 46.

The end effector slider 47 has a first bifurcated main body 79 with a tubular front connection piece 80 that allows the tube 3 to pass through its lumen to reach the junction slider 48 where it is mounted so that the junction slider can slide all of the tube 3, the reinforcing coil member 77, the effector sleeve 21, the end effector 21 in combination and simultaneously.

The reinforcing coil member 77 is connected to a tubular front connection piece 80 of the end effector slider 47 and oppositely to the effector sleeve 21 to reciprocate the effector sleeve 21 to open and close the jaws 19,20.

The first spring member 55 can be positioned between opposite first legs 81,82 of the first bifurcated main body 79 to aid in spring-biasing any of the sliders 47,48,49. The junction slider 48 can move in the lengthwise extending opening 85 of the tubular guide housing 50 of the main slide guide body 46 towards the distal guide end 53 of said main slide guide body 46 between the first legs 81,82 of the end effector slider 47 until it hits on the end stop 83 constituted by the distal end 84 of the lengthwise extending opening 85 of the main slide guide body 46. The exterior wall 86 of the end effector slider 47 has opposite lengthwise extending protrusions 87 that slide in the interior tracks 51 of the tubular guide housing 50 of the main slide guide body 46. A first operating string securing arm 88 protrudes from the end effector slider 47 close to the tubular front connection piece 80 in a plane below the first bifurcated main body 79. The first operating string securing arm 88 has a first eye or hole 89 for securing of or passage of a first operating string 116 connected to a trigger button 125 of the remote operating handle 120 of the remote actuator assembly 6, as will be explained in further details with references to figs. 11 and 12.

The junction slider 47 has a slide part 90 and protruding first sliding shoes 91 of dimensions selected to control and facilitate smooth sliding of the junction slider 48 along the interior tracks 51 and along the guideway 54. The junction slider 47 can also slide between the first legs 81,82 of the end effector slider 47. The junction slider 48 further has a main junction body 92 with a through-going axially extending bore 93 that receives the tube 3 so that the first lengthwise extending channel 9 is put in fluid communication with an inlet port 94 of the junction slider 48, and the second lengthwise extending channel 10 is put in liquid communication with the sample port 72 of the junction slider 48. The slide part 90 may be a front part of the main junction body 92. The opposite first sliding shoes 91 protrude from the main junction body 92 to slide in the interior tracks 51. A bearing 119 for a sheave member 73 of a sheave assembly 78 protrudes from the main junction body 92 through the lengthwise extending opening 85 of the main slide guide body 46. The bearing 119 has a coupling hole 121 for attaching a sheave assembly 78 of the remote actuator assembly 6, e.g. attaching a shaft or pin. The sheave member may be rotationally arranged in the bearing 119 or be stationary.

The electrical diathermy wire 76 runs inside the third lengthwise extending channel 15 and is externalized via a diathermy port 95 of the junction slider 47, which diathermy port 95 is further configured for plugging in an electrical plug (not shown) of the diathermy generator (not shown).

The through-going bore 93 extends lengthwise through the main junction body 92 inside the fourth lengthwise extending channel 16 of the tube 3 to allow the needle 22 to pass through to reach the needle slider 49.

A second operating string securing arm 96 protrudes from the junction slider 48 in a plane below the main junction body 92. The second operating string securing arm 96 has a second eye of hole 97 for guiding the operating strings of the remote actuator assembly 6, which is seen in fig. 12.

The needle slider 49 is also bifurcated in that it has a second bifurcated main body 98 constituted by opposite second legs 99,100 that join in an injection port 101 that have liquid access to the needle 22. The second legs 99,100 is configured to grasp around the sample port 72 of the junction slider 48, to allow the sample port 72 to slide in between said second legs 99,100.

In order to couple to the junction slider 48 and the needle slider 49, the tube 3 may be divided in its respective sub-tubes with the first lengthwise extending channel 9 turned in communication with the inlet port 94 of the junction slider 48, the second lengthwise extending channel 10 is turned in communication with the sample port 72 of the junction slider 48, the third lengthwise extending channel 15 is turned in communication with the diathermy port 95 of the junction slider 48, and the fourth lengthwise extending channel 16 is directed into or towards the injection port 101 of the needle slider 49 via the junction slider 48. The fourth lengthwise extending channel 16 of the tube 3 may seal around the needle 22 that runs inside the fourth lengthwise extending channel 16 of the tube 3. The needle 22 alone, or the fourth lengthwise extending channel 16 and the needle 12, may continue into the injection port 101. Other arrangements of obtaining liquid communication between the injection port 101 and the needle 22 are within the scope of the present invention. Medicament may be injected into the needle 22 via the injection port 101, e.g. by using a hypodermic needle to penetrate a membrane (not shown) of the injection port.

The needle slider 49 has a protruding curved flexible clamp 102 that conforms around the exterior wall of the tubular guide housing 50 of the main slide guide body 46. Thus the needle slider 49 may be slidingly snapped on the main slide guide body 46. The protruding curved flexible clamp 102 also has a third eye of hole 104 below the second bifurcated main body 98 to also serve as a securing arm 103 for a third operating string 118 to put the needle slider 49 in operatively communication with the remote actuator assembly 6, thus to move the needle 22 in and out of the end effector 22.

A detachable cover or plug 105 can open and close the proximal guide end 52 of the main slide guide body 46 to mount the sliders 47,48,49 and the spring members 55,56 in the guideway 54. The detachable cover or plug 105 may have an indent or similar coupling component 106 to mate firmly with mating means 107 at a free holder end 108 of the holder 30. To that aspect the end 109 of the holder 30 opposite the free holder end 108 has a hole 110 for inserting the tube 3 and for guiding said tube 3 into the instrument port of the cystoscope 2 via the adaptor 28

The tubular guide housing 50 has a fourth eye 111 at the proximal guide end 52 and a fifth eye 112 at the opposite distal guide end 53.

The sheave assembly 78 includes a sheave member 113 having a first sheave track 114 and a second sheave track 115, a first operating string 116, a second operating string 117, and a third operating string 118. The sheave member 113 is suspended in the bearing 119 of the junction slider 48 and extends through the lengthwise extending opening 85 of the tubular guide housing 50 of the main slide guide body 46 to keep the second operating string 117 under control and free of entangling with the first operating string 116 and the third operating string 118.

The second operating string 117 has a second distal string end 117a secured to the fifth eye 112 at the distal guide end 53 of the tubular guide housing 50 of the main slide guide body 46. The second operating string 117 then runs around the first track 114 of the sheave member 113 of the sheave assembly through the second eye 97 of the junction slider 48 further inside the guide tube 122, such as a Bowden conduit, and is fixed to the wheel button 125, such as a thumb wheel or roller, of the remote operating handle 120. From its fixation point at the wheel button 125 the second operating string 117 returns again inside the guide tube 122 via a second track 115 of the sheave member 113 of the sheave assembly 78 to have its second proximal string end 117b secured to a fourth eye 111 at the proximal guide end 52 of the tubular guide housing 50 of the main slide guide body 46. The second operating string 117 can then reciprocate the junction slider 48 to move the operating end 131 of the endosurgical biopsy device 1 of the present invention, thus the distal tube end 13 with the end effector 21 surrounded by the effector sleeve 17, in and out of the distal opening of the working channel of the cystoscope to position said operating end towards the target site.

This arrangement of the second operating string 117 minimizes slack and play, and the required dimension of guide tube 122, which e.g. is a Bowden conduit, is much smaller than if it would have been used in compression, thereby avoiding buckling of the guide tube 122 and trapping of the operating strings 116,117,118 extending therein.

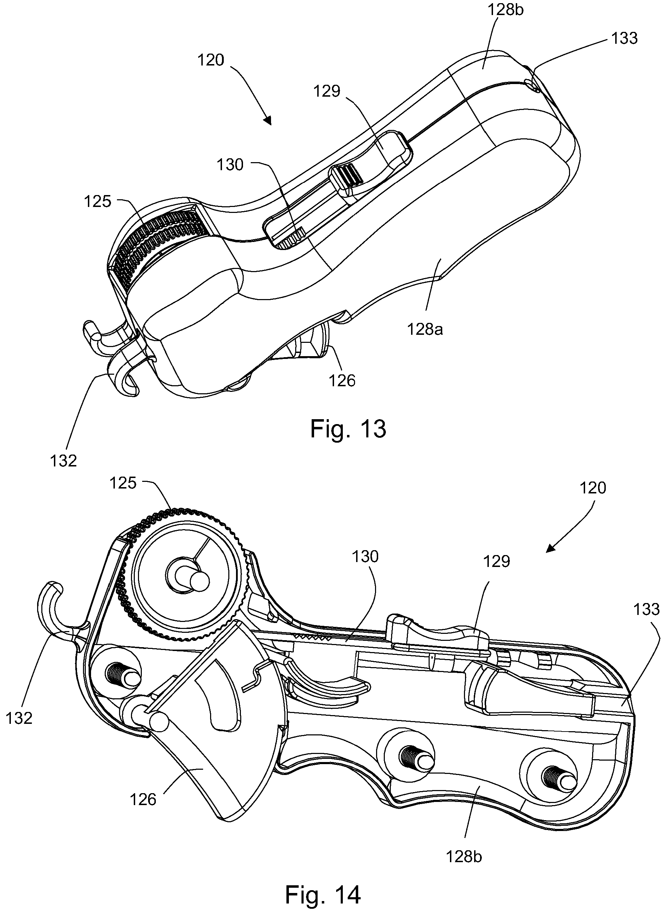

The remote operating handle 120 of the remote actuator assembly 8 is seen in perspective oblique from the side, and in the interior view of fig. 14 of the same a casing-half 128a of the opposite casing halves 128a,128b of a casing 128 of the remote operating handle 120 has been left out to visualize the arrangement of the trigger button 126, the wheel button 125 and the slider button 129 and the operating strings 116,117,118. The casing 128 has a snap-on means 132 at it foremost end to snap the remote operating handle 120 to the distal tube part of the cystoscope 2. Opposite the snap-on means 132 the casing has en entry for the tube guide 122. The snap-on means 132 is suited to detachable snap the remote operating handle 120 on to the distal tube part of the cystoscope 2 perpendicularly protruding therefrom. Further or other snap-on means can be provided otherwise on the casing to facilitate other snap-on orientations of the remote operating handle 120.

The wheel button 125 may have its rotating shaft spring-suspended. In its normal position the wheel button 125 is locked against the casing 128 of the remote operating handle 120 by the wheel button 125 engaging the casing 128. When pushing the wheel button 125 down again in its operating opening 127 the axis of the rotating shaft shifts and the wheel button 125 releases from its engagement with the casing 128 and can rotate again. The locking arrangement of the wheel button 125 in relation to the casing 128, inside and free of its operating opening 127, is seen in enlarged scale view in fig. 12. The remote operating handle is seen in assembled state in fig. 13.

None of the spring members 55,56 are involved to expose the operating end 131 of the endosurgical biopsy device 1 of the present invention from the working channel of the cystoscope 2.

A first operating string 116 has a first distal string end 116b secured to the trigger button 126 at the remote operating handle 120. The first operating string 116 extends via the guide tube 122 towards its proximal string securing end 116a to be attached to the end effector slider 47, e.g. to the first eye 89. Moving the end effector slider 47 backwards by actuating the trigger button 126 pulls the effector sleeve free of the end effector 17, and compresses the first spring member 55 against the junction slider 48, thereby allowing the jaws 19,20 of the end effector 21 to spring apart. When releasing the trigger button 126, the spring force of the first spring member 55 closes the jaws 19,20 of the end effector with a limited force to avoid damage to the end effector 21 thereby ensuring repeatable conditions for the electrosurgical resecting sequence.

The end effector can be made from two pipe pieces each been given a tapered or blunt nose. The pipe pieces can be cut from separate pipes, e.g. by laser cutting lengthwise and providing the flexible members 19a,20a, the hinge members 19a,20a. The pipe pieces are subsequently joined e.g. to obtain a main tubular body with spring-biased hinged jaws thereby obtaining the end effector 21. After cutting the respective hinge members 19a,20a can deflect to curve away from each other. Thus the hinge members 19a,20a make the end effector jaws resilient and flexible in relation to each other. The respective hinge members 19a,20a can deflect by itself by inherent property given to them by outwards bending of the jaws to curve away when the effector sleeve is not covering the end effector, thus the end effector opens and closes resiliently.

A third operating string 118 has a third proximal string end 118a secured to the third eye 104 of the needle slider 49, and an opposite third distal string end 118b secured to a slider button 129 of the remote operating handle 120 to reciprocate the needle slider 49 between the opposite jaws 19,20. When the slider button 129 is moved forward towards the junction slider the second spring member 56 is compressed, which places a spring force on the needle slider that makes the needle 22 to retract by retracting the needle slider 49 once the slider button releases the compression force on the second spring member 56.

To control when the actions of the slider button 129, and thus release of the compression force of the second spring member 55, the slider button 129 may have a ratchet extension 130 facing the trigger button 126. The ratchet extension can engage a mating ratchet on the trigger button 126 to interlock the slider button 129 and the trigger button 126 so that the trigger button 126 does not unintentionally releases the spring force of the first spring member 55 and not unintentionally pushes the effector sleeve 17 forward to close the jaws 19,20 while the needle is still exposed between said jaws 19,20.

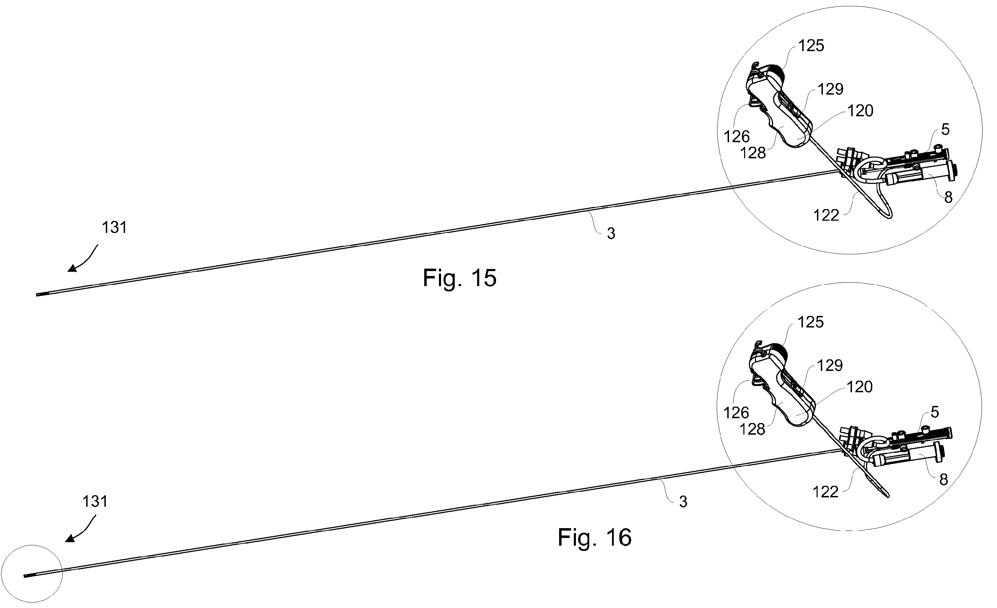

Fig. 15 shows the endosurgical biopsy device in its starting position. None of the spring members 55,56 are biased, the end effector 21 is inside the effector sleeve 17, and the wheel button 125 is locked to the casing 128 in its operating opening 127.

In the situation seen in fig. 16 the wheel button 125 has been unlocked and rotated to advance the operating end of the endosurgical biopsy device 1 from the distal end of the cystoscope (not shown). During this advancing the junction slider 48 moves forward due to the second operating string's 117 attachment to the sheave member 73 of the junction slider 48. The needle 22, which is connected to the needle slider 49 slides inside or along the junction slider 48 when the junction slider moves in the guideway 54.

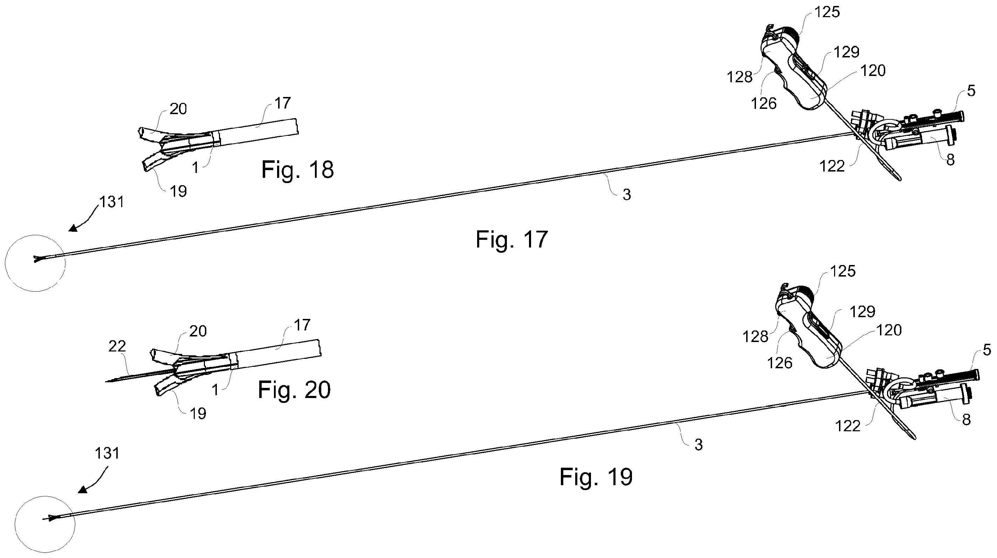

In the situation seen in fig. 17 the trigger button 126 has been actuated to expose the end effector 21 from the effector sleeve 17 whereby the opposite jaws 19,20 of the end effector 21 open. When depressing the trigger button 126 the first operating string 116 is pulled at, and the first operating string 116 pulls the end effector slider 47 towards the proximal guide end 52. The junction slider is thereby in a position at least partly between the opposite legs 81,82 of the end effector slider 47, which end effector slider 47 retracts the reinforcing coil to which the effector sleeve is mounted end-to-end, thereby leaving the end effector 21 free and the opposite jaws 19,20 to inherently open, as seen in the enlarged scale view of fig. 18 of the operating end 131 of the endosurgical biopsy device 1.

Due to the securing of the third operating string 118 to both the slider button 129 and the needle slider 49, the needle slider 49 can be moved towards the junction slider 48 by sliding the slider button 129 forward, and thereby moving the needle 22 out between and beyond the open jaws 19,20, as seen in figs. 19 and 20 to make an injection. To make sure that the jaws do not close unintentionally while the needle 22 is exposed the slider button 129 and the trigger button 126 engages during the injection.

The needle is retracted by moving the slider button in the opposite direction; the jaws are then closed around the tissue by releasing the trigger button to move the effector sleeve forward. And diathermy is applied, e.g. by operating a foot pedal of the diathermy generator to resect the tissue specimen.

Regarding the end effector 21, said end effector has opposite jaws 19,20 arranged to diverge from a longitudinal axis of the end effector in a relaxed condition when the end effector 21 is at least partly outside the effector sleeve 17, due to flexible hinge members 19a,20a.

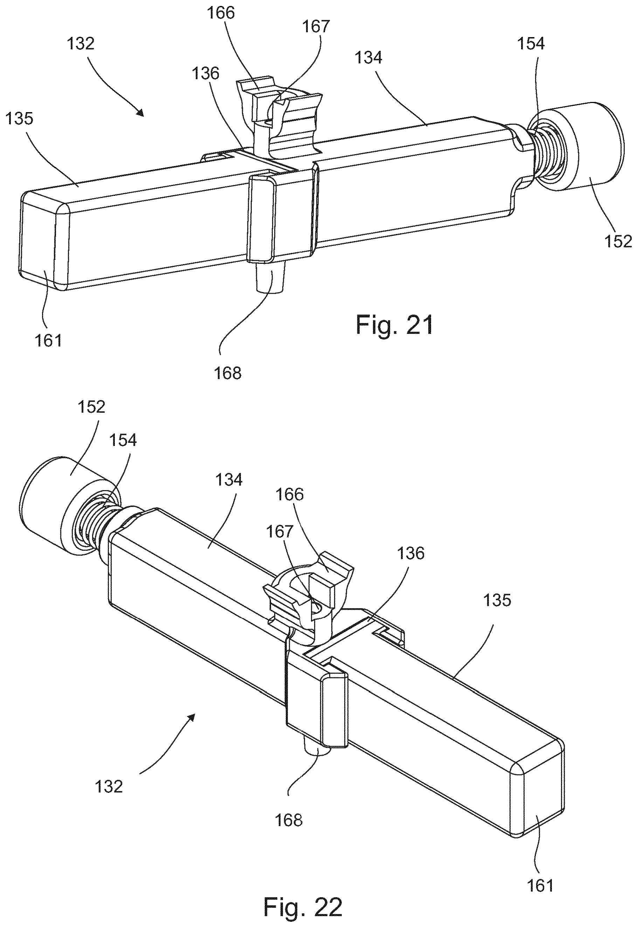

Figs. 21 and 22 show in perspective a second embodiment of a tissue specimen collector 132 according to the present invention from opposite ends. The functionality of the second embodiment of a tissue specimen collector 132 corresponds to the functionality of the first embodiment of a tissue specimen collector 8. The mechanism that arrange the chambers of the collector rack below the sample port of the rack assembly is however different.

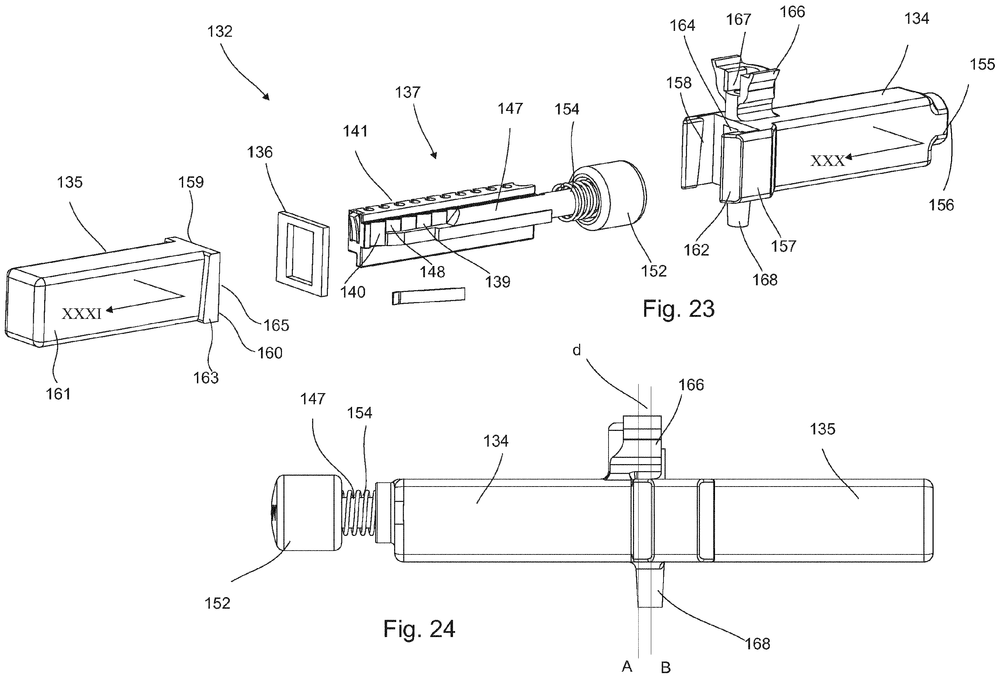

The main collector housing 133 of the second embodiment of a tissue specimen collector 132 consist of two detachably assembled tubular housing parts, a proximal tubular housing part 134 which is assembled with a distal tubular housing part 135 with a seal 136 or gasket in-between those housing parts 134,135, as seen best in the exploded view of fig. 23.

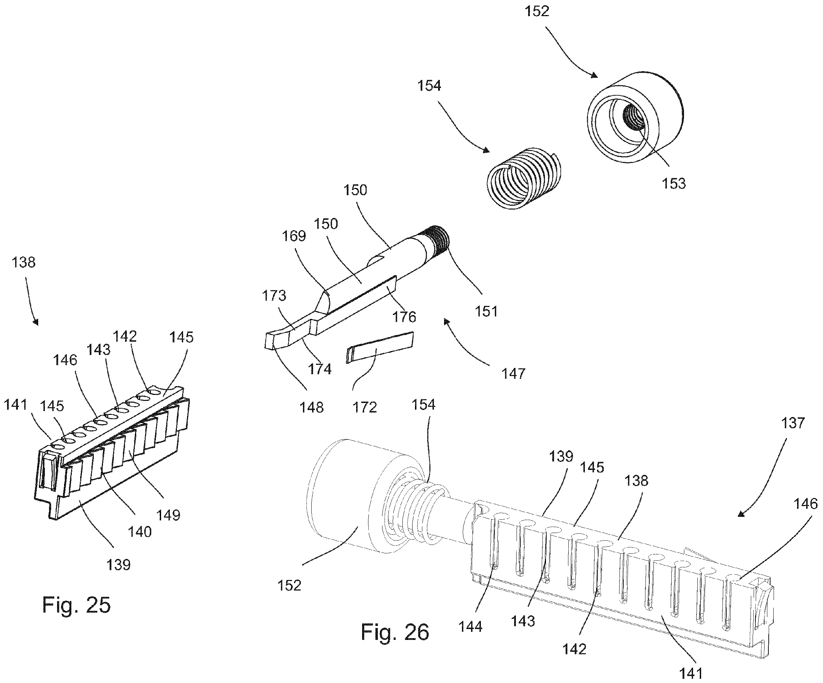

The collector rack assembly, which is also seen in more details in the enlarged scale perspective view of figs. 26 - 28, and in perspective exploded view in fig. 25, includes a spring-biased ratchet mechanism 137. The spring-biased ratchet mechanism includes a collector rack 138 that has a first lengthwise extending face 139 with a linear rack 140, and an opposite extending lengthwise extending face 141 into which drainage canals 142 discharge liquid from the dedicated chambers 143 into the main collector housing 133, which chambers 143 has closed bottoms 144 so that the tissue specimens do not pass through. For the same reason the width of the drainage canals 142 are smaller than the diameter of chambers 143, such as e.g. half the diameter or less than the diameter of the chambers 143.

A top surface 145 of the collector rack 138 has inlet openings 146 for each of the dedicated chambers 143. In the second embodiment of a collector rack 138 of the collector rack assembly seen in perspective assembled view in figs. 26 - 28, and in exploded perspective view in fig. 25, the collector rack 138 has ten dedicated chambers 143. In the configuration of the collector rack 138 seen in figs. 26 - 28, the collector rack 138 has its foremost chamber 143a below and aligned with the coupling piece 166 of the proximal tubular housing part 134.

The collector rack assembly further includes a pawl member 147 arranged reciprocatingly alongside the linear rack 140 and having a pawl tip 148 to stepwise engage any tooth 149 of the teeth of the linear rack 140, which tooth of the teeth depends on the axial position of the collector rack 138 inside the respective tubular housing parts 134,135, which collector rack is driven stepwise forward inside the distal tubular housing part 135 by means of application of a pressure force to the pawl member 147.

The pawl tip 148 extends into a push rod part 150 towards a proximal threaded push rod part 151. The proximal threaded push rod part 151 serves for securing an actuator button 152, which actuator button has an interior actuator button threading 153 that mate the threading on the proximal threaded push rod part 151. A third spring member 154 surrounds the proximal threaded push rod part 151 so that when the actuator button 152 has been depressed towards the distal tubular housing part 135 the third spring member 154 is compressed, and the collector rack moves one step forward along the length of the collector rack to place the next chamber in the series of chambers below the collector port. Next the force applied to the third spring member 154 is relieved making the push rod part 150 to return to its starting position thereby placing the pawl tip 148 behind the next tooth of the linear rack 140 to be moved forward.

As seen best in fig. 23 the proximal tubular housing part 134 has a first proximal housing end 155 with a proximal housing opening 156 opposite a first distal housing end 157 with a distal housing opening 158. The distal tubular housing part 135 has a second proximal housing end 159 with a proximal housing opening 160 opposite a second distal housing end 161, which is blind.

The first distal housing end 157 has a first engagement means 162, and the second proximal housing end 159 has a second engagement means 163, that engagingly mates the first engagement means 162 to firmly secure the proximal tubular housing part 134 and the distal tubular housing part 135 to each other. The seal 136 ensures a leak-tight, preferably detachable, securing together of the tubular housing parts 134,135.

In the second embodiment of a tissue specimen collector 132 the first engagement means 162 is female and the second engagement means 163 is male. Within the scope of the present invention the arrangement of engagement means can be opposite, so that the male engagement means is on the first distal housing end 157 and the female engagement means is on the second proximal housing end 159. In the exemplary embodiment of engagement means shows e.g. in fig. 23 the first engagement means 162 is in form of a dove-tail track for receiving a mating second engagement means 163 in form of a dove-tailed key, e.g. by slidingly force-fitting the dove-tailed key inside the dove-tail track to hold the tubular housing parts 134,135 frictionally together and unable to be pulled from each other. Within the scope of the present invention the arrangement of engagement means can be otherwise, such as clamped together, screwed together, etc.

While the proximal tubular housing part 74 and the distal tubular housing part 75 of the first embodiment of a tissue specimen collector 8 have different interior cross-sections, circular and square, respectively, the proximal tubular housing part 134 and the distal tubular housing part 135 of the second embodiment of a tissue specimen collector 138 both have generally square internal cross-sections of their respective tubular bores 164,165 to slidingly accommodate a substantially square collector rack.

It is preferred, although not mandatory, that at least the main collector housing 133 is transparent to let the surgeon monitor the tissue specimen-containing flow inside the tissue specimen collector 132, and to monitor that the tissue specimens actually are received inside the chambers 143 one after the other in each their respective chamber 143.

The proximal tubular housing part 134 has a coupling piece 166 adapted to couple together with e.g. a tubing or a sample port of an endosurgical biopsy device or instrument, such as e.g. coupling to the sample port 72 of the main junction body 92 of the junction slider 48 of the slide rail assembly seen and described in relation to figs. 1 - 21.

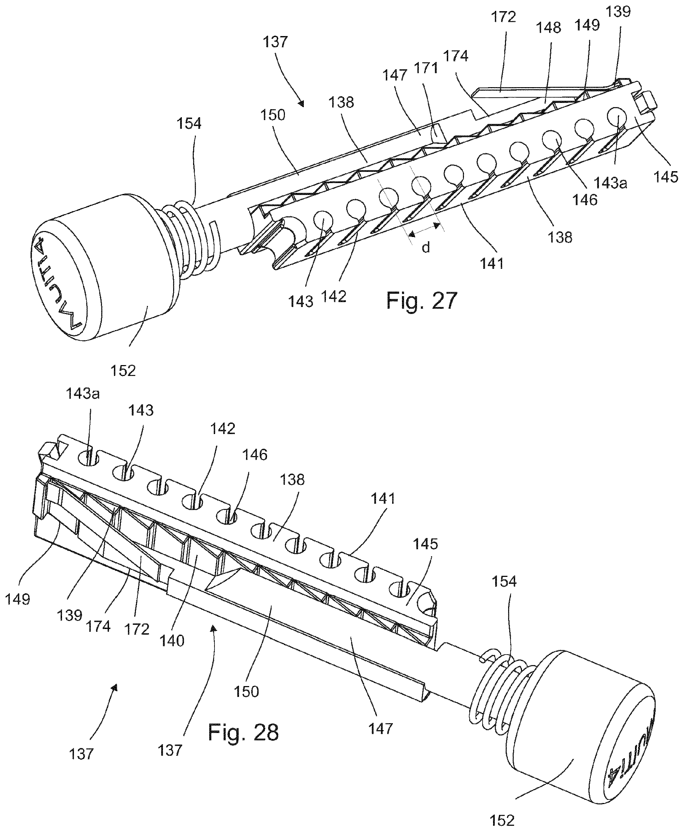

The coupling piece 166 protrudes from a collector port 167 of the proximal tubular housing part 134 opposite a liquid outlet 168 arranged for discharging liquid from the chambers 143 of the collector rack 137, optionally also discharging any liquid inside the main collector housing 133. As seen best in the side view of fig. 24 the centre axis A of the liquid outlet 168 is lengthwise axially offset the centre axis B of the collector port a distance d corresponding to the distance d between the centers of two adjacent chambers 143, as illustrated in fig. 27, so that when the collector rack is moved forward by the pawl member 137 engaging a tooth of the linear rack 141 a new chamber 143 is moved below the collector port 167, and the drainage canal 142 of the chamber 143 that accommodate the newest tissue specimen is simultaneously moved above and in alignment with the liquid outlet 168, whereby any liquid in at least said chamber 143 with the last arrived tissue specimen can be drained directly into a reservoir or other receptacle.

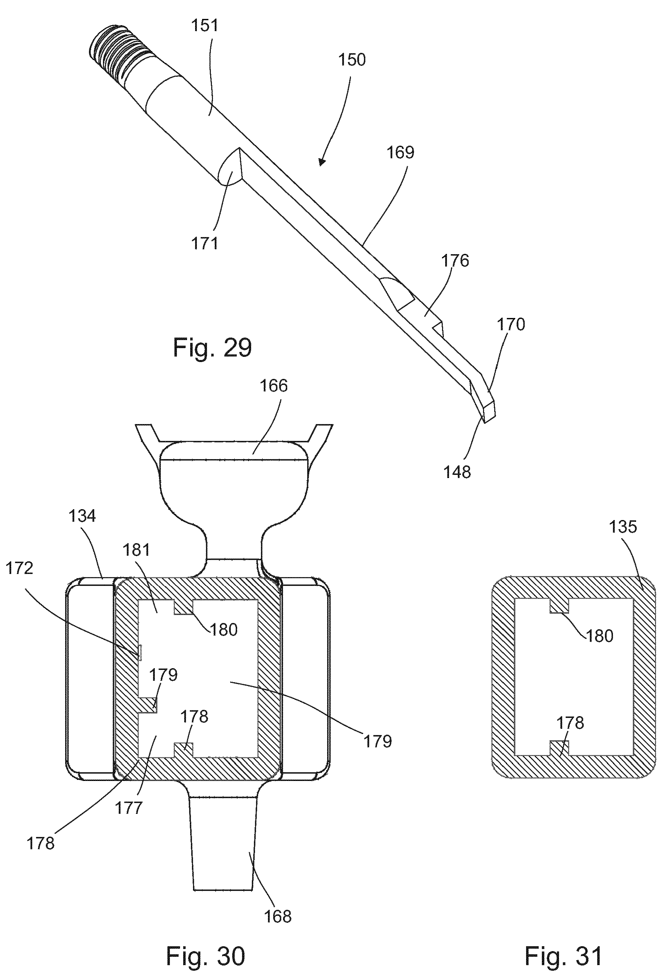

As seen best in figs. 25 and 29 the push rod part 150 has a distal push rod part 169 opposite the proximal threaded push rod part 151, where the pawl tip 148 is at the free end 170 of the distal push rod part 169.

The distal push rod part 169 protrudes from the proximal threaded push rod part 151 with a reduced cross-section lengthwise from said distal push rod part 169 thereby exposing an off centre stop face 171 at the transition 171 between the distal push rod part 169 and the proximal threaded push rod part 151. The stop face 171 defines the maximum possible stroke length of the push rod part 150 towards the collector rack 138, in that when the push rod part 150 is moved against the collector rack the stop face 171 hits on the proximal end of the collector rack 138. In this way the possible maximum travel of the push rod part 150 can e.g. be delimited to a travel of one tooth of the linear rack. Optionally said travel is thus the maximum possible depression of the push rod part 150. Such depression can be tactile so that when the surgeon feels the engagement between the free end 170 of the distal push rod part 169 and the next tooth along the linear rack he/she positively knows that engagement has taken place, and that a new chamber is aligned below the sample port. The longitudinal axial length of a tooth of the linear rack may allows a small freedom of travel of the push rod part 150 due to said length of the teeth, which preferably may be angled towards the push rod part 151 to improve engagement. The stop face 171 advantageously limits unintentional over-depression during a stroke of the push rod part 151.

A leaf spring 172 may advantageously be inserted resiliently between the interior face 175 of the proximal tubular housing part 135 and the free end part 173 of the distal push rod part 169 to apply a radial force on the free end 170 of the distal push rod part 169 against a tooth of the linear rack to prevent accidental dislocation of the alignment of a chamber below the sample port. The free end part 173 of the distal push rod part 169 may have a lengthwise extending recess 174 to accommodate the flexing of the leaf spring 172 when it is becomes pressed against the wall of the proximal tubular housing part 134.

As seen in the sectional view of fig. 29 a guide rib 176 protrudes radially from the distal push rod part 169 lengthwise between the recess 174 and the stop face 171 to slide in a first housing track 177 along the bottom 178 of at least the proximal tubular housing part 134, as illustrated in fig. 30, to allow the linear rack 138 to slide confined in a second housing track 179 adjacent the first housing track 177. The first housing track is delimited by a bottom protrusion 179 from the bottom 178 and a substantially perpendicularly extending protrusion 180 a distance above the bottom 178 that provides sufficient confined space for the guide rib 176 to slide. The second housing track 179 is delimited by the bottom protrusion 179 and a top protrusion 180 from the interior wall of the proximal tubular housing part 134 opposite the upright protrusion 179.

The second housing track 179 opens into a third housing track 181 that allows the free end 170 of the distal push rod part 169 to jump above the teeth of the linear rack 138 to engage one tooth of the liner rack after the other until all chambers are filled with a respective tissue specimen.

The distal tubular housing part 135, which is seen inside in the cross sectional view of fig. 31, has a similar bottom protrusion 179 and top protrusion 180 in extension of same of the proximal tubular hosing part 134 so that the linear rack can slide inside the distal tubular housing part 125 when it is moved forward by the push rod part 150. Any of the housing tracks are advantageously dimensioned to accommodate its corresponding slide body, the linear rack and the guide rib so that these objects does not skew when slid.

Alternative means than lengthwise extending protrusions suited to divide the tubular housing parts lengthwise into appropriate housing tracks are within the scope of the present invention. **E.g.** can a housing part have the female mating part and the linear rack and the push rod part have the male mating part.

The multi-functional endosurgical biopsy device of the present invention suggests a whole new way to take biopsies from a hollow organ or tissue surface. Completely new conditions are created for the treatment of these patients. The costs can be drastically reduced and the patient benefit is extremely high. It will be possible to diagnose and correct outpatients with cancers in a few minutes on a regular visit. Patients no longer need any catheter treatment after surgery, which means they eliminate the high risk of urinary tract infection that a catheter entails. All different categories of staff no longer need to be involved; it is enough with a doctor and nurse, which means a huge cost reduction, and patients can receive cancer treatment much faster, and surgical capacity for other purposes are released.

It is emphasized that the tissue specimen collectors of the present invention can be used with any instrument or device than can be coupled to a tube, e.g. in connection with surgical needle biopsy devices, with or without using endoscopes. The organ from which the tissue specimen is taken can be others than the urinary bladder, such as the lungs, the stomach, the intestines, the vagina, etc.

Further the usability of the tissue specimen collector of the present invention is not limited to uses when coupled to a tube. In fact the tissue specimen collector of the present invention can be used as a stand alone unit and the tissue specimens placed in the dedicated chambers manually using syringes, tweezers or forceps. So although the latter uses reduce the demand on reduced risk of contamination, the cassette concept can anyway be used simply to keep a series of specimens collected in same unit under combined identification data.

The tissue specimen collector of the present invention may conveniently be adapted for collecting tissue specimens during a biopsy procedure. The tissue specimen collector may be a multi-chamber collector having a main collector housing, a collector rack arranged lengthwise displaceable inside the main collector housing, wherein the collector rack can have a series of adjacent chambers for accommodating tissue specimens. Said main collector housing may conveniently have a collector port adapted to connect to a supply of tissue specimens to receive tissue specimens in each consecutive chamber of the serious of adjacent chambers in the order they are resected from suspicious tissue.

## Claims

1. A tissue specimen collector (8;132) adapted for collecting tissue specimens during a biopsy procedure, wherein the tissue specimen collector (8;132) is a multi-chamber collector having
- a main collector housing (58;133) extending along a longitudinal axis and comprising a collector port (71a;167) adapted to connect tc a supply of tissue specimens,
wherein that the multi-chamber collector has
- a collector rack (59;138) having a series of adjacent chambers (60;143) for accommodating tissue specimens, which collector rack (59;138) is arranged lengthwise displaceable inside the main collector housing (58;133) to move translatory inside said main collector housing (58;133) by actuating means for moving the collector rack (59;138) lengthwise along the longitudinal axis of the main collector housing (58;133) to position said chambers (60;143) one after another in fluid communication with the collector port (71a;167) **characterized in that** said collector housing is configured to keep all of the tissue specimens in a closed environment simultaneously

2. A tissue specimen collector (8;132) according to claim 1, wherein the tissue specimen collector forms a single multi-chamber cassette or cartridge configured to collect said tissue specimens.

3. A tissue specimen collector (8;132) according to any of claims 1 or 2, **characterized in that** the tissue specimen collector (8;132) include a coupling piece (71;166) adapted for providing fluid coupling between the collector port (71a;167) and a sample port (72) of an endosurgical biopsy device (1).

4. A tissue specimen collector (8;132) according to claim 3, wherein the coupling piece (71;166) protrudes from the collector port (71a;167)) of the main collector housing (58;133).

5. A tissue specimen collector (8;132) according to any of claims 1-4, **characterized in that** the main collector housing (58;133) has a liquid outlet (68;168)) to discharge liquid accompanying the tissue specimen entering the tissue specimen collector (8;132).

6. A tissue specimen collector (8) according to any of claims 1-5, **characterized in that** the means (61) for moving the collector rack (59) lengthwise along the longitudinal axis of the main collector housing (58) is a screw rod (62) that engages with a threaded hole (63) or bore in the collector rack (59), optionally the screw rod (62) has a rotating knob (64) at a free end (65) facing away from the collector rack (59).

7. A tissue specimen collector (132) according to any of claims 1-6, **characterized in that** the means (137) for moving the collector rack (138) lengthwise along the longitudinal axis of the main collector housing (133) is ratchet mechanism consisting of at least a linear rack (140) on the collector rack (138) and a pawl member (147), preferably a spring-biased pawl member, that stepwise engages the linear rack (140) of the collector rack (138).

8. A tissue specimen collector (8;132) according to any of the preceding claims 1 - 7, **characterized in that** the collector rack (59;138) is configured to allow liquid to pass through, optionally the collector rack (59;138) is a filtering unit, optionally a chamber (60;143) has a drainage canal (142) that discharge into the main collector housing (58;133) or the wall of the collector rack has one or more drainage channels.

9. A tissue specimen collector (8;132) according to any of the preceding claims 1 - 8, **characterized in that** the main collector housing (58;133) is detachable assembled of at least two housing parts (74,75;134,135).

10. A tissue specimen collector (8;132) according to claim 9, further comprising a seal or gasket (136) between said at least two housing parts (74,75;134,135).

11. An endosurgical biopsy device (1) comprising:
- a tube (3) that has at least a first lengthwise extending channel (9) for supplying a liquid to the end effector (21), a second lengthwise extending channel (10) for removing matter from or at the end effector (21), and a third lengthwise extending channel (15) for accommodating an electrical wire (76) connected to the end effector (21) to apply current to perform diathermy,
- an end effector (21) provided at a distal tube end (13) of the tube, and
a tissue specimen collector (8;132) according to any of the preceding claims 1 - 10 provided in fluid communication with a proximal tube end of the tube.

12. An endosurgical biopsy device (1) according to claim 11,
**characterized in that** the endosurgical biopsy device (1) comprises
- an effector sleeve (17) surrounding the tube (3) at least at the distal tube end (14) of said tube (3), and being arranged to reciprocate at least at the distal tube end (13) of the tube (7),
- a device operating part (4) comprising a slide rail assembly (5), which slide rail assembly (5) comprises at least a main slide guide body (46) that accommodates an end effector slider (47) and a junction slider (48),
- the main slide guide body (46) has a guideway (54) with a proximal guide end (52) and an opposite distal guide end (53) that receives the tube (3),
- the end effector slider (47) is adapted to reciprocatingly slide in the guideway (54) at the distal guide end (53), and is connected to the effector sleeve (17) to operate said effector sleeve (17) in relation to the end effector, and
- the junction slider (48) is adapted to reciprocatingly slide in the guideway (54) between the proximal guide end (52) and the end effector slider (47), which junction slider (48) is connected to the tube (3) to arrange at least the first lengthwise extending channel (9), the second lengthwise extending channel (10) and the third lengthwise extending channel (15) of the tube (3) in communication with the end effector (21).

13. An endosurgical biopsy device (1) according to any of claims 11-12, **characterized in that** the slide rail assembly (5) further has a needle slider (49) reciprocatingly disposed in the guideway (54) in front of the junction slider (48) at the proximal guide end (52), which needle slider (49) has a needle (22) or a nozzle secured thereto, which needle (22) or nozzle is reciprocatingly arranged inside a fourth lengthwise extending channel (16) of the tube (3) between a first needle position in which the needle or nozzle is in a retracted position and a second needle position in which the needle or nozzle is exposed from the end effector (21).

14. An endosurgical biopsy device (1) according to any of claims 12 or 13, **characterized in that** the junction slider (48) has at least one of
- an inlet port (94) in fluid communication with the first lengthwise extending channel (9) for delivering a flushing liquid at and/or to the end effector (21),
- a diathermy port (95) in communication with the third lengthwise extending channel (15), and/or
- a sample port (72) in communication with the second lengthwise extending channel (10) for coupling with the tissue specimen collector (8;132).

15. An endosurgical biopsy device (1) according to any of claims 12-14, **characterized in that** the device operating part (4) further comprise a flushing component (7) having a first operating end part (18) adapted to be mounted to an endoscope fluid valve port and to an endoscope suction valve port of an endoscope (2), and an opposite second operating end part (27) adapted to be set in fluid communication with the slide rail assembly (5) and with an instrument port of the endoscope (2).

## Patentansprüche

1. Gewebeprobensammelvorrichtung (8; 132), die so ausgelegt ist, dass sie während einem Biopsieverfahren Gewebeproben sammelt, wobei die Gewebeprobensammelvorrichtung (8; 132) eine Mehrkammer-Sammelvorrichtung ist, die Folgendes aufweist:
- ein Hauptsammelvorrichtungsgehäuse (58; 133), das sich eine Längsachse entlang erstreckt und eine Sammelvorrichtungsöffnung (71a; 167) umfasst, die so ausgelegt ist, dass sie mit einer Zufuhrvorrichtung für Gewebeproben verbunden wird,
wobei die Mehrkammer-Sammelvorrichtung Folgendes aufweist:
- einen Sammelvorrichtungsrahmen (59; 138) mit einer Reihe von benachbarten Kammern (60; 143) zum Aufnehmen von Gewebeproben, wobei der Sammelvorrichtungsrahmen (59; 138) längs verschiebbar in dem Hauptsammelvorrichtungsgehäuse (58; 133) angeordnet ist, damit er sich geradlinig in dem Hauptsammelvorrichtungsgehäuse (58; 133) bewegt, indem Mittel zum Bewegen des Sammelvorrichtungsrahmens (59; 138) längs entlang der Längsachse des Hauptsammelvorrichtungsgehäuses (58; 133) betätigt werden, damit die Kammern (60; 143) nacheinander in Fluidverbindung mit der Sammelvorrichtungsöffnung (71a; 167) gebracht werden, **dadurch gekennzeichnet, dass** das Sammelvorrichtungsgehäuse so eingerichtet ist, dass sämtliche Gewebeproben gleichzeitig in einer geschlossenen Umgebung bleiben.

2. Gewebeprobensammelvorrichtung (8; 132) nach Anspruch 1, wobei die Gewebeprobensammelvorrichtung eine einzelne Mehrkammerkassette oder -kartusche bildet, die so eingerichtet ist, dass sie die Gewebeproben sammelt.

3. Gewebeprobensammelvorrichtung (8; 132) nach einem der Ansprüche 1 oder 2, **dadurch gekennzeichnet, dass** die Gewebeprobensammelvorrichtung (8; 132) ein Verbindungsstück (71; 166) aufweist, das so ausgelegt ist, dass es für eine Fluidkopplung zwischen der Sammelvorrichtungsöffnung (71a; 167) und einer Probenöffnung (72) einer endochirurgischen Biopsievorrichtung (1) sorgt.

4. Gewebeprobensammelvorrichtung (8; 132) nach Anspruch 3, wobei das Verbindungsstück (71; 166) aus der Sammelvorrichtungsöffnung (71a; 167) des Hauptsammelvorrichtungsgehäuses (58; 133) ragt.

5. Gewebeprobensammelvorrichtung (8; 132) nach einem der Ansprüche 1-4, **dadurch gekennzeichnet, dass** das Hauptsammelvorrichtungsgehäuse (58; 133) eine Flüssigkeitsaustrittsöffnung (68; 168) zum Ablassen von Flüssigkeit aufweist, die mit der Gewebeprobe einhergeht, die in die Gewebeprobensammelvorrichtung (8; 132) gelangt.

6. Gewebeprobensammelvorrichtung (8) nach einem der Ansprüche 1-5, **dadurch gekennzeichnet, dass** das Mittel (61) zum Bewegen des Sammelvorrichtungsrahmens (59) längs entlang der Längsachse des Hauptsammelvorrichtungsgehäuses (58) eine Gewindestange (62) ist, die in eine Gewindebohrung (63) oder Bohrung im Sammelvorrichtungsrahmen (59) eingreift, die Gewindestange (62) optional einen Drehknopf (64) an einem freien Ende (65) auf weist, der von dem Sammelvorrichtungsrahmen (59) weg weist.

7. Gewebeprobensammelvorrichtung (132) nach einem der Ansprüche 1-6, **dadurch gekennzeichnet, dass** das Mittel (137) zum Bewegen des Sammelvorrichtungsrahmens (138) längs entlang der Längsachse des Hauptsammelvorrichtungsgehäuses (133) ein Sperrmechanismus ist, der aus mindestens einer geraden Zahnstange (140) an dem Sammelvorrichtungsrahmen (138) und einem Sperrklinkenelement (147), vorzugsweise einem federbelasteten Sperrklinkenelement, besteht, das schrittweise in die gerade Zahnstange (140) von dem Sammelvorrichtungsrahmen (138) eingreift.

8. Gewebeprobensammelvorrichtung (8; 132) nach einem der vorhergehenden Ansprüche 1-7, **dadurch gekennzeichnet, dass** der Sammelvorrichtungsrahmen (59; 138) so eingerichtet ist, dass Flüssigkeit hindurchfließen darf, der Sammelvorrichtungsrahmen (59; 138) optional eine Filtereinheit ist, eine Kammer (60; 143) optional einen Ablaufkanal (142) aufweist, der in das Hauptsammelvorrichtungsgehäuse (58; 133) abläuft, oder die Wand des Sammelvorrichtungsrahmens einen oder mehrere Abläufkanäle aufweist.

9. Gewebeprobensammelvorrichtung (8; 132) nach einem der vorhergehenden Ansprüche 1-8, **dadurch gekennzeichnet, dass** das Hauptsammelvorrichtungsgehäuse (58; 133) lösbar aus mindestens zwei Gehäuseteilen (74, 75; 134, 135) zusammengesetzt ist.

10. Gewebeprobensammelvorrichtung (8; 132) nach Anspruch 9, ferner umfassend eine Dichtung (136) zwischen den mindestens zwei Gehäuseteilen (74, 75; 134, 135).

11. Endochirurgische Biopsievorrichtung (1), umfassend:
- einen Schlauch (3), der mindestens einen ersten sich längs erstreckenden Kanal (9) zum Zuführen einer Flüssigkeit zum Endeffektor (21), einen zweiten sich längs erstreckenden Kanal (10) zum Entfernen von Material von oder an dem Endeffektor (21) und einen dritten sich längs erstreckenden Kanal (15) zum Aufnehmen eines elektrischen Drahts (76) aufweist, der mit dem Endeffektor (21) verbunden ist und Strom zum Durchführen einer Diathermie liefert,
- einen Endeffektor (21), der an einem distalen Schlauchende (13) des Schlauchs vorgesehen ist, und
- eine Gewebeprobensammelvorrichtung (8; 132) nach einem der vorhergehenden Ansprüche 1-10, die in Fluidverbindung mit einem proximalen Schlauchende des Schlauchs vorgesehen ist.

12. Endochirurgische Biopsievorrichtung (1) nach Anspruch 11, **dadurch gekennzeichnet, dass** die endochirurgische Biopsievorrichtung (1) Folgendes umfasst:
- eine Effektorhülse (17), die den Schlauch (3) zumindest am distalen Schlauchende (14) des Schlauchs (3) umgibt und so angeordnet ist, dass sie sich zumindest am distalen Schlauchende (13) des Schlauchs (7) hin- und herbewegt,
- einen Vorrichtungsbetätigungsteil (4), der eine Gleitschienenanordnung (5) umfasst, wobei die Gleitschienenanordnung (5) mindestens einen Gleitführungshauptteil (46) umfasst, der ein Endeffektorgleitelement (47) und ein Verbindungsgleitelement (48) aufnimmt,
- der Gleitführungshauptteil (46) eine Führungsbahn (54) mit einem proximalen Führungsende (52) und einem gegenüberliegenden distalen Führungsende (53) aufweist, die den Schlauch (3) aufnimmt,
- das Endeffektorgleitelement (47) so ausgelegt ist, dass es sich in der Führungsbahn (54) am distalen Führungsende (53) hin- und herbewegt, und mit der Effektorhülse (17) so verbunden ist, dass die Effektorhülse (17) relativ zu dem Endeffektor betätigt wird, und
- das Verbindungsgleitelement (48) so ausgelegt ist, dass es sich in der Führungsbahn (54) zwischen dem proximalen Führungsende (52) und dem Endeffektorgleitelement (47) hin- und herbewegt, wobei das Verbindungsgleitelement (48) mit dem Schlauch (3) so verbunden ist, dass zumindest der erste sich längs erstreckende Kanal (9), der zweite sich längs erstreckende Kanal (10) und der dritte sich längs erstreckende Kanal (15) des Schlauchs (3) mit dem Endeffektor (21) verbunden angeordnet ist.

13. Endochirurgische Biopsievorrichtung (1) nach einem der Ansprüche 11-12, **dadurch gekennzeichnet, dass** die Gleitschienenanordnung (5) ferner ein Nadelgleitelement (49) aufweist, das sich hin- und herbewegend in der Führungsbahn (54) vor dem Verbindungsgleitelement (48) am proximalen Führungsende (52) angeordnet ist, wobei das Nadelgleitelement (49) eine daran befestigte Nadel (22) oder Düse aufweist, wobei die Nadel (22) oder Düse in einem vierten sich längs erstreckenden Kanal (16) des Schlauchs (3) so angeordnet ist, dass sie sich zwischen einer ersten Nadelstellung, in der sich die Nadel oder Düse in einer zurückgezogenen Stellung befindet, und einer zweiten Nadelstellung, in der die Nadel oder Düse aus dem Endeffektor (21) ragt, hin- und herbewegt.

14. Endochirurgische Biopsievorrichtung (1) nach einem der Ansprüche 12 oder 13, **dadurch gekennzeichnet, dass** das Verbindungsgleitelement (48) Folgendes aufweist:
- eine Einlassöffnung (94) in Fluidverbindung mit dem ersten sich längs erstreckenden Kanal (9) zum Zuführen einer Spülflüssigkeit an das und/oder zum Endeffektor (21),
- einen Diathermieanschluss (95) in Verbindung mit dem dritten sich längs erstreckenden Kanal (15) und/oder
- eine Probenöffnung (72) in Verbindung mit dem zweiten sich längs erstreckenden Kanal (10) zum Koppeln mit der Gewebeprobensammelvorrichtung (8; 132).

15. Endochirurgische Biopsievorrichtung (1) nach einem Ansprüche 12-14, **dadurch gekennzeichnet, dass** der Vorrichtungsbetätigungsteil (4) ferner einen Spülbestandteil (7) mit einem ersten Betätigungsendteil (18), der so ausgelegt ist, dass er an einem Endoskop-Fluidventilanschluss und an einem Endoskop-Saugventilanschluss eines Endoskops (2) montiert wird, und einem gegenüberliegenden zweiten Betätigungsendteil (27) umfasst, der so ausgelegt ist, dass er mit der Gleitschienenanordnung (5) und mit einem Instrumentenanschluss des Endoskops (2) in eine Fluidverbindung gebracht wird.

## Revendications

1. Collecteur d'échantillon de tissu (8 ; 132) adapté pour la collecte d'échantillons de tissu pendant une biopsie, dans lequel le collecteur d'échantillon de tissu (8 ; 132) est un collecteur multi-chambres ayant
- un logement de collecteur principal (58 ; 133) s'étendant le long d'un axe longitudinal et comprenant
- un port de collecteur (71a ; 167) adapté pour se raccorder à une alimentation en échantillons de tissu,
dans lequel le collecteur multi-chambres présente
- un bac de collecteur (59 ; 138) ayant une série de chambres adjacentes (60 ; 143) pour loger des échantillons de tissu, lequel bac de collecteur (59 ; 138) est agencé en longueur en étant mobile à l'intérieur du logement de collecteur principal (58 ; 133) pour se déplacer en translation à l'intérieur dudit logement de collecteur principal (58 ; 133) en déclenchant des moyens pour déplacer le bac de collecteur (59 ; 138) en longueur le long de l'axe longitudinal du logement de collecteur principal (58 ; 133) pour positionner lesdites chambres (60 ; 143) l'une après l'autre en communication fluidique avec le port de collecteur (71a ; 167), **caractérisé en ce que** ledit logement de collecteur est configuré pour maintenir l'ensemble des échantillons de tissu dans un environnement fermé simultanément.

2. Collecteur d'échantillon de tissu (8 ; 132) selon la revendication 1, dans lequel le collecteur d'échantillon de tissu forme une cassette ou cartouche multi-chambres unique configurée pour collecter lesdits échantillons de tissu.

3. Collecteur d'échantillon de tissu (8 ; 132) selon l'une quelconque des revendications 1 ou 2, **caractérisé en ce que** le collecteur d'échantillon de tissu (8 ; 132) inclut une pièce de couplage (71 ; 166) adaptée pour fournir un couplage fluidique entre le port de collecteur (71a ; 167) et un port d'échantillon (72) d'un dispositif de biopsie endochirurgicale (1).

4. Collecteur d'échantillon de tissu (8 ; 132) selon la revendication 3, dans lequel la pièce de couplage (71 ; 166) fait saillie du port de collecteur (71a ; 167) du logement de collecteur principal (58 ; 133).

5. Collecteur d'échantillon de tissu (8 ; 132) selon l'une quelconque des revendications 1-4, **caractérisé en ce que** le logement de collecteur principal (58 ; 133) présente une sortie de liquide (68 ; 168) pour décharger du liquide accompagnant l'échantillon de tissu entrant dans le collecteur d'échantillon de tissu (8 ; 132).

6. Collecteur d'échantillon de tissu (8) selon l'une quelconque des revendications 1-5, **caractérisé en ce que** le moyen (61) pour déplacer le bac de collecteur (59) en longueur le long de l'axe longitudinal du logement de collecteur principal (58) est une tige filetée (62) qui s'engage avec un trou (63) ou alésage fileté dans le bac de collecteur (59), en option, la tige filetée (62) présente une molette rotative (64) à une extrémité libre (65) orientée à distance du bac de collecteur (59).

7. Collecteur d'échantillon de tissu (132) selon l'une quelconque des revendications 1-6, **caractérisé en ce que** le moyen (137) pour déplacer le bac de collecteur (138) en longueur le long de l'axe longitudinal du logement de collecteur principal (133) est un mécanisme à encliquetage consistant en au moins un rail linéaire (140) sur le bac de collecteur (138) et un membre de cliquet (147), de préférence un membre de cliquet sollicité par ressort, qui engage pas à pas le rail linéaire (140) du bac de collecteur (138).

8. Collecteur d'échantillon de tissu (8 ; 132) selon l'une quelconque des revendications 1-7, **caractérisé en ce que** le rail de collecteur (59 ; 138) est configuré pour permettre à du liquide de passer au travers, en option, le rail de collecteur (59 ; 138) est une unité de filtrage, en option une chambre (60 ; 143) présente un canal de drainage (142) qui décharge dans le logement de collecteur principal (58 ; 133) ou bien la paroi du bac de collecteur présente un canal ou des canaux de drainage.

9. Collecteur d'échantillon de tissu (8 ; 132) selon l'une quelconque des revendications 1-8, **caractérisé en ce que** le logement de collecteur principal (58 ; 133) est séparable en étant assemblé d'au moins deux parties de logement (74, 75 ; 134, 135).

10. Collecteur d'échantillon de tissu (8 ; 132) selon la revendication 9, comprenant en outre une garniture ou un joint (136) entre lesdites aux moins deux parties de logement (74, 75 ; 134, 135).

11. Dispositif de biopsie endochirurgicale (1) comprenant :
- un tube (3) qui présente au moins un premier canal (9) s'étendant en longueur pour fournir un liquide à l'effecteur terminal (21), un deuxième canal (10) s'étendant en longueur pour retirer de la matière de ou au niveau de l'effecteur terminal (21), et un troisième canal (15) s'étendant en longueur pour loger un fil électrique (76) raccordé à l'effecteur terminal (21) pour appliquer du courant pour effectuer une diathermie,
- un effecteur terminal (21) prévu à une extrémité de tube distale (13) du tube, et
un collecteur d'échantillon de tissu (8 ; 132) selon l'une quelconque des revendications précédentes 1 - 10 prévu en communication fluidique avec une extrémité de tube proximale du tube.

12. Dispositif de biopsie endochirurgicale (1) selon la revendication 11, **caractérisé en ce que** le dispositif de biopsie endochirurgicale (1) comprend
- un manchon d'effecteur (17) entourant le tube (3) au moins à l'extrémité de tube distale (14) dudit tube (3) et étant agencé pour effectuer un va-et-vient au moins à l'extrémité de tube distale (13) du tube (7),
- une partie opérationnelle de dispositif (4) qui comprend un ensemble de rail coulissant (5), lequel ensemble de rail coulissant (5) comprend au moins un corps principal de guidage coulissant (46) qui loge une coulisse d'effecteur terminal (47) et une coulisse de jonction (48),
- le corps principal de guidage coulissant (46) présente une voie de guidage (54) avec une extrémité proximale de guidage (52) et une extrémité distale de guidage (53) opposée qui reçoit le tube (3) ;
- la coulisse d'effecteur terminal (47) est adaptée pour coulisser en va-et-vient dans la voie de guidage (54) à l'extrémité distale de guidage (53), et est connectée au manchon d'effecteur (17) pour faire fonctionner ledit manchon d'effecteur (17) par rapport à l'effecteur terminal, et
- la coulisse de jonction (48) est adaptée pour coulisser en va-et-vient dans la voie de guidage (54) entre l'extrémité proximale de guidage (52) et la coulisse d'effecteur terminal (47), laquelle coulisse de jonction (48) est raccordée au tube (3) pour agencer au moins le premier canal (9) s'étendant en longueur, le deuxième canal (10) s'étendant en longueur et le troisième canal (15) s'étendant en longueur du tube (3) en communication avec l'effecteur terminal (21).

13. Dispositif de biopsie endochirurgicale (1) selon l'une quelconque des revendications 11-12, **caractérisé en ce que** l'ensemble de rail coulissant (5) présente en outre une coulisse d'aiguille (49) disposée en va-et-vient dans la voie de guidage (54) devant la coulisse de jonction (48) à l'extrémité proximale de guidage (52), laquelle coulisse d'aiguille (49) présente une aiguille (22) ou une buse fixée dessus, laquelle aiguille (22) ou buse est agencée en va-et-vient à l'intérieur d'un quatrième canal (16) s'étendant en longueur du tube (3) entre une première position d'aiguille et une seconde position d'aiguille dans laquelle l'aiguille ou la buse est exposée de l'effecteur terminal (21).

14. Dispositif de biopsie endochirurgicale (1) selon l'une quelconque des revendications 12 ou 13, **caractérisé en ce que** la coulisse de jonction (48) présente au moins l'un de
- un port d'entrée (94) en communication fluidique avec le premier canal (9) s'étendant en longueur pour fournir un liquide de rinçage au niveau du et/ou à l'effecteur terminal (21),
- un port de diathermie (95) en communication avec le troisième canal (15) s'étendant en longueur (15), et/ou
- un port d'échantillon (72) en communication avec le deuxième canal (10) s'étendant en longueur pour le couplage avec le collecteur d'échantillon de tissu (8 ; 132).

15. Dispositif de biopsie endochirurgicale (1) selon l'une quelconque des revendications 12-14, **caractérisé en ce que** la pièce fonctionnelle de dispositif (4) comprend en outre une composante de rinçage (7) ayant une première partie terminale opérationnelle (18) adaptée pour être montée sur un port de valve fluidique d'endoscope et sur un port de valve d'aspiration d'endoscope d'un endoscope (2), et une seconde partie terminale opérationnelle (27) opposée adaptée pour être en communication fluidique avec l'ensemble de rail coulissant (5) et avec un port d'instrument de l'endoscope (2).
